# EUROPÄISCHE PATENTSCHRIFT

(11) **EP 1 513 492 B1**
(45) Veröffentlichungstag und Bekanntmachung des Hinweises auf die Patenterteilung: **24.01.2007**
(21) Anmeldenummer: 03740198.1
(22) Anmeldetag: 06.06.2003
(51) Int. Cl.: A61K 8/06, A61K 8/64, A61Q 19/08, A61Q 19/04, A61Q 19/02

(54) **MIKRO-EMULSIONEN MIT BINÄRER PHASEN- UND WIRKSTOFFDIFFERENZIERBARKEIT, DEREN HERSTELLUNG UND DEREN VERWENDUNG, INSBESONDERE ZUR TOPISCHEN SAUERSTOFFVERSORGUNG**
MICROEMULSIONS HAVING A BINARY PHASE DIFFERENTIABILITY AND ACTIVE SUBSTANCE DIFFERENTIABILITY, THE PRODUCTION THEREOF AND THEIR USE, PARTICULARLY FOR THE TOPICAL SUPPLY OF OXYGEN
MICROEMULSIONS PRESENTANT UNE DIFFERENTIABILITE DE PHASE BINAIRE ET DE SUBSTANCE ACTIVE, LEUR PRODUCTION ET LEUR UTILISATION EN PARTICULIER POUR ADMINISTRER DE L'OXYGENE PAR VOIE TOPIQUE

(30) Priorität: 18.06.2002 DE 10226990
(43) Veröffentlichungstag der Anmeldung: 16.03.2005
(73) Patentinhaber: SanguiBioTech GmbH, 58455 Witten an der Ruhr (DE)
(72) Erfinder: BARNIKOL, Wolfgang, 55128 Mainz (DE); TESLENKO, Alexander, 58095 Hagen (DE)
(74) Vertreter: Müller, Claudia
(86) Internationale Anmeldenummer: PCT/EP2003/005948
(87) Internationale Veröffentlichungsnummer: WO 2003/105797

(56) Entgegenhaltungen:
- EP-A- 0 753 311
- WO-A-01/91728
- WO-A-02/05754
- WO-A-92/18147
- FR-A- 2 741 266

## Beschreibung

### Gegenstand der Erfindung

Die vorliegende Erfindung betrifft hautverträgliche zur Haar- und Hautbehandlung geeignete Mikro- Emulsionen auf Basis einer primären W/O-Mikro- Emulsionen, die sowohl in eine sekundäre W/O als auch in eine sekundäre O/W- Mikro- Emulsion überführt werden und insbesondere sowohl wasserlösliche als auch fettlösliche Wirkstoffe in stabiler Form enthalten kann. Bevorzugt enthält die Emulsion einen Sauerstoffbinder wie Hämoglobin, mit welchem gebundener bioverfügbarer Sauerstoff, vorzugsweise zusammen mit weiteren Wirkstoffen durch topische Anwendung in die Haut eingebracht werden kann, um das Zellwachstum des Stratum germinativums zu unterstützen. Diese Emulsionen können einfach ohne technischen Aufwand hergestellt und sowohl in der Kosmetik als auch in der Medizin (Dermatologie) angewendet werden.

### Stand der Technik

Mikro-Emulsionen sind makroskopisch homogene, optisch isotrope und thermodynamisch stabile 2-phasige Systeme, die aus zwei nicht mischbaren Flüssigkeiten (in der Regel Wasser und aus einer oder mehreren unpolaren nicht mit Wasser mischbaren organischen Flüssigkeiten, allgemein als "Öl" bezeichnet) bestehen. Weiterhin enthalten sie Tenside. Im einfachsten Fall bilden sich Mikro-Emulsionen bereits aus diesen drei Komponenten, häufig ist jedoch ein Cotensid (z.B. ein kurzkettiger aliphatischer Alkohol) erforderlich. Sie können als kontinuierliche Phase Wasser (O/W-Mikro- Emulsion) oder Öl als kontinuierliche Phase (W/O-Mikro- Emulsion) haben.

Mikro-Emulsionen besitzen die einzigartige Eigenschaft, dass die Oberflächenspannung zwischen den Phasen sehr gering ist. Dadurch sind die Phasen thermodynamisch gegen Phasen-Trennung sehr stabil; somit sind die Mizellen in Form sehr kleiner Partikel mit einer Größe von 20 bis 200 nm (im Gegensatz zu üblichen Emulsionen mit einer Partikelgröße von 1 bis 20 µ) existenzfähig/P.Kumar, K.L.Mittal (Eds) Handbuck of Microemulsions. Marcel Dekker Inc. N.Y., 1999/.

Die bekannten Mikro- Emulsionen werden je nach Wirkstoffeigenschaft formuliert. Sie haben ein breites Anwendungsgebiet, wie die Erdölgewinnung, der Bereich technischer und häuslicher Reinigungsmittel, sowie die Feindispersion chemischer Substanzen, beispielsweise Pestizide und Fungizide u.a.m. Ein weiteres potentielles Einsatzgebiet ist ihre Verwendung als Reaktionsmedium für chemische oder enzymatische Reaktionen zwischen wasserlöslichen Reaktanden und unpolaren organischen Verbindungen.

Mikro-Emulsionen sind zudem ein ideales Transport-Vehikel für die perorale, intrakutane oder transkutane Gabe von Medikamenten für Menschen und Tiere:

So werden in US- B 6 113 921 O/W-Mikro-Emulsionen beschrieben, die 0,5 bis 30 Gew. % einer Ölphase zusammen mit 0,05 bis 5 % eines nichtionischen Tensids, 0,1 bis 10 % eines Emulgators, wie Phospholipide und wasserunlösliche Wirkstoffe wie Diclophenac, enthalten. Durch Anwendung des Hochdruck-Homogenisators APV-Gaulin bei einem Druck von etwa 800 bar und bei erhöhter Temperatur weisen die Partikel eine Größe von 10 bis 500 nm auf.

Es ist ferner ein Verfahren zur Herstellung von Zusammensetzungen bekannt, wobei eine Mischung von Glycerid-Tensiden mit einem HLB- Wert ≤ 16 und Propylenglykol- oder Polyglycerolestern mit einer Öl- und einer Wasserphase und einem Wirkstoff gemischt werden. Bei Kontakt mit einer biologischen Flüssigkeit, z.B. Magensaft, entsteht in situ eine Mikro- Emulsion , vgl. US 6 309 665.

Die US-B 5,646,109 (EP-B 0746 331) betrifft konvertierbare oral oder intravenös anzuwendende W/O- Mikro- Emulsionen mit einem wasserlöslichen Wirkstoff und bis zu 70 % eines Tensidgemisches aus mindestens einem C₉-₁₃-Monoglycerides mit einem HLB- Wert von ≤ 8 und einem Tensid mit einem HLB- Wert von ≥ 8.

EP-B 0580 778 beschreibt eine W/O- Emulsion mit einem wasserlöslichen Wirkstoff und Tensiden mit HLB- Werten von 7 bis 14, wobei zwingend ein C₇₋₅₅-Propylenglykoldiester in der Ölphase enthalten ist.

Die WO 02/09671 offenbart zur Emulgierung von wasserunlöslichen pharmakologischen Wirkstoffen (Analgetika, Antidepressiva, Immunosuppressiva, Antineoptastika u.a.) eine Mischung aus Poloxamer Block Copolymeres mit C₈₋₁₂-Fettsäuren wie Natriumlaurat. Gemäß der WO 00 06 690 Mikro-Emulsionen auf der Basis von kationischen Tensiden für die Anwendung im Bereich Haarpflege beschrieben.

Chang II Hong et al., US B 6,063,762 und US B 6306 434 haben eine Cyclosporin enthaltende pharmazeutische Komposition für die orale Anwendung beschrieben. Hier wird Cyclosporin mit einem Öl und einem Tensid vermischt und sodann trocken mit einem Polycarbonat oder Polyol (Eudragit® )umgesetzt.

US- B 6 191 105 beschreibt eine Zusammensetzung zur oralen Verabreichung eines Insulin- Hexansäure- Konjugates zur Behandlung von Diabetes. Hierbei ist ein O/W- Tensid in der Wasserphase und 30 bis 90 % einer Ölphase vorhanden.

Ferner wurden Mikro-Emulsionen und Mizellen intravenös angewendet, u. a. für die Behandlung von Gehim-Tumoren, vgl. WO 02109671.

Nach der US-A 6,191,105 werden intramuskulär verabreichbare Zusammensetzungen für die gentherapeutische Behandlung verschiedener Erkrankungen beschrieben, wobei Polynukleotide wie RNA und DNA in Form einer wässrigen Dispersion in weniger als 0,1 % Poloxamer- Polyacrylat- Copolymeren vorliegen.

Im Vergleich zu diesen pharmazeutischen Mikro-Emulsionen sollten die für kosmetische Zwecke entwickelten Mikro-Emulsionen keine systemische Wirkung erzielen, d. h. sie sollten lediglich penetrieren bis zur basalen Membran des Hautepithels oder bis zur oberen Schicht der Dermis. Diese Hautschicht, das Stratum corneum, wird gebildet von abgestorbenen Keratinocyten, die sich im Laufe der Zeit abschuppen. Der gesamte Prozess von der Mitose bis zum Absterben der Zellen und ihrer Abschuppung beträgt normalerweise etwa 4 Wochen. Die Hornschicht (Stratum corneum) trägt zur die Intaktheit der Haut wegen ihrer Barriere-Funktionen entscheidend bei.

Mit zunehmendem Alter verlangsamt sich der mitotische Prozess, nach heutiger Erkenntnis schon ab dem 30-sten Lebensjahr. Dies führt zur Bildung einer immer dünneren Haut mit verringerter Lebensfähigkeit. Die Hautalterung ist zum einen genetisch bedingt. Ferner wirken sich Unterversorgung mit Nähr- und Baustoffen sowie zahlreiche Umwelteinflüsse, insbesondere Sonnenlicht, negativ auf die Haut aus Eine entscheidende Rolle spielt hierbei der Sauerstoff.

Um die Versorgung der Haut zu unterstützen sollten die oben genannten Stoffe bis zu den Zellen des Hautepithels geliefert werden. Voraussetzung hierfür ist die topische Zugänglichkeit zu den vitalen Zellen des Hautepithels. Während die Schleimhäute lokaltherapeutisch direkt erreichbar sind, werden die vitalen Zellen des Hautepithels von außen durch die schwer durchdringliche Hornschicht von 10-20 µm Dicke geschützt. Soll ein Wirkstoff zu den vitalen Zellen des Hautepithels gelangen, so kann dies nur mit Hilfe eines geeigneten Vehikels geschehen, das die Hornhaut-Barriere zu überwinden gestattet. Man unterscheidet hierbei mindestens zwei Schritte, nämlich
(1) Penetration: Eintritt einer Substanz in das Stratum corneum
(2) Permeation: Die Diffusion einer Substanz vom Stratum corneum in die lebende Epidermis.

Diese Prozesse, die ebenso für die oben beschriebene medizinische Anwendung topischer Mittel bedeutsam sind, müssen sowohl bei der kosmetischen Hautpflege, nämlich der Stärkung der natürlichen Funktion der Haut als Barriere gegen Umwelteinflüsse und gegen den Verlust von körpereigenen Stoffen (z.B. Wasser, natürliche Fette) als auch bei der kosmetischen Behandlung, nämlich der Unterstützung der vitalen Zellen mit essentiellen Nährstoffen und somit der Wiederherstellung der funktionsschwachen Bestandteile der Haut, z.B. durch Förderung der Kollagen- und Hyaluronsäure - Biosyntese, ablaufen.

Hierzu wendet man neben oftmals technisch aufwendige mehr oder weniger wirksame physikalische Methoden wie Ultraschall, Elektroporese oder lontophorese, traditionelle kosmetische Präparate wie Cremes, Lotionen, Gele und Liposome an.

Diesen gegenüber haben Mikro- Emulsionen den Vorteil der kleineren Teilchengröße, also der besseren Permeation und der geringeren erforderlichen Wirkstoffmenge und sei sind stabiler, insbesondere im Vergleich zu liposomalen Produkten.

Es sind eine Reihe von Mikro-Emulsionen in der Kosmetik bekannt:

Nach der WO 98/15254 (EP 0930 866) werden W/O- Mikro-Emulsionsgele für verschiedene kosmetische Zubereitungen wie Lotionen, Duschlotionen, "Aftershave"-Lotionen, Deospray, Anti-Akne-Gele, Sonnenschutzmittel (UV-Filters),deodorierende Stoffe, wasserfestes . Augen-Make-up und andere kosmetische Zubereitungen auf Basis von W/O-Emulgatoren beschrieben, die zwingend Vernetzer wie Dimethicon- Copolyole enthalten.

Die US-B 6 315 989 betrifft eine Wasserstoffperoxid enthaltende als W/O - Mikro-Emulsion Komposition für die Färbung von Haaren, die neben der Öl- und der Wasserphase 1-65 % eines organischen Tensids mit einem HLB- Wert von 12-16 aufweist.

In den Patenten US-B 6,207,140, US-B 5,876,702 und der EP-A 1 092 414 werden UV-Filter -haltige Mikro-Emulsionen für den Schutz der Haut vor Sonnenstrahlen beschrieben, die insbesondere pH- oder Temperatur- abhängige lipophil veränderliche Emulgatoren aufweisen. Dabei werden insbesondere sulfonierte UV-Filter eingesetzt, welche als Elekrolyten wirken und somit ebenfalls die Lipophilie der Emulgatoren beeinflussen.

Im Patent US- B 5 389 607 werden alkoholfreie parfümhaltige Mikro-Emulsionen mit einem auf Polyethylenglykol basierenden Tensid zusammen mit einem Polyglycerol und einem Phosphatether beschrieben.

Aus diesem Stand der Technik ist ersichtlich, dass die topische Anwendung von Mikro-Emulsionen in erster Linie auf eine Wirkung auf der Hautoberfläche abzielt. Die oben beschriebenen Mikro- Emulsionen zeigen keine tiefe Penetration in die Hornschicht oder Dermis. Darüber hinaus sind diese entweder kompliziert herstellbar (z. B. nur mit hohem Druck oder in situ), oder es sind sehr hohe Tensidanteile erforderlich. Schließlich wirken bereits beschriebene Emulsionen nur auf Schleimhäuten.

Die DE A1 44 11 557 betrifft ein Verfahren zur Herstellung von W/O- Emulsionen, indem als Ölkomponente mindestens 30 % eines C₁₂₋₂₄-Dialkylethers, 10 bis 35 % eines lipophilen Emulgators mit HLB- Wert 6 bis 10 (W/O-Emulgator) und 1 bis 10 % eines hydrophilen (O/W) Emulgators mit HLB- Wert > 11 eingesetzt werden. Somit liegt immer ein Überschuss an W/O zu O/W- Emulgator in Verbindung mit Dialkylethern als Ölkomponente vor.

In der WO 94/26234 werden W/O- Emulsionen beschrieben, welche zwingend 25 bis 85 % Petrolatum und 15 bis 40 % feste Wachse sowie maximal 5 % Wasser aufweisen, um als Lippenbalsamformulierungen eingesetzt werden zu können.

Dementsprechend werden die Formulierungen in der Wärme hergestellt, damit das Wachs ausreichend geschmolzen werden kann.

Die US- A 4,797,273 betrifft eine W/O- Mirko- Emulsion, die zwingend eine Polysiloxanverbindung aufweist. Dies soll insbesondere bei trockener Haut wirksam sein.

In der US- A 4,797,272 werden übliche W/O- Mikro- Emulsionen zur Feuchthaltung der Haut beschrieben mit üblichen Tensiden und verschiedenen Feuchthaltekomponenten.

In der WO A 00/61083 werden transparente Mikro- Emulsionen offenbart, wobei das Verhältnis von Tenside(n) zu Ölkomponente(n) 2:1 bis 1:1 beträgt. Die Zusammensetzung wird unter Anwendung von Wärme (75° bis 80° C) hergestellt.

### Aufgabe der Erfindung

Aufgabe vorliegender Erfindung ist es daher, ein Mittel bereitzustellen, mit weichem die Haut oder Haar von außen mit den notwendigen zellulären Nährstoffen in ausreichender Menge wirksam versorgt werden kann, wobei insbesondere eine Versorgung mit Sauerstoff erfolgen und die Hornschicht als Barriere überwunden werden soll. Dabei soll keine Beschränkung auf nur wasserlösliche oder nur öl-(fett-) lösliche Wirkstoffe vorhanden sein.

Ferner soll das Mittel einfach herstellbar und die Tensidmenge möglichst gering sein.

Ein weiterer Zweck der Erfindung ist es, mittels derartiger Mittel insbesondere degenerative, durch äußere oder immunologisch bedingte Einflüsse beschädigte oder veränderte Haut wirksam zu behandeln, sowohl kosmetisch als auch dermatologisch/ pharmazeutisch.

Insbesondere soll eine Versorgung mit Sauerstoff derart erfolgen, dass es zu keiner überhöhten oder schädlichen Versorgung wie z.B. bei gasförmig angewandtem Sauerstoff kommt, welche sich toxisch auswirken kann. Hierzu gehört auch eine wirksame Penetration, welche wie erwähnt mit normalen Emulsionen nicht möglich ist, vgl. die aus der US-B 5 380 764 bekannte Zusammensetzung aus Vitamin, Glukose und Wasserstoffperoxyd in Form einer O/W-Emulsion als Creme, wobei hier neben der schlechten Penetration noch - das - Problem des physikalisch wirkenden Sauerstoffs, der das Gewebe schädigen kann, s.o. besteht.

Das Mittel soll ferner leicht anwendbar sein als solches und daher insbesondere flüssig oder gelförmig zum Einreiben, insbesondere aber auch fein sprühbar sein, ohne dass jedoch hierfür spezielle Substanzen wie Gelbildner erforderlich wären.

### Lösung der Aufgabe

Diese Aufgaben werden erfindungsgemäß gelöst, indem eine Mikro- Emulsion gemäß Anspruch 1 bereitgestellt wird. Überraschenderweise weisen derartige Mikro- Emulsion Nano-Mizellen auf, ohne dass bestimmte Vernetzer wie z.B. Dimethicon erforderlich wären. Darüber hinaus können gleichzeitig sowohl wasserlösliche als auch fettlösliche Wirkstoffe inkorporiert werden, ohne dass es zu einer Instabilität käme. Schließlich kann die so bereitete Mikro- Emulsion durch Umsetzung mit einer Wasserphase in eine sekundäre W/O- Mikro-Emulsion oder bei einem erhöhten Wassergehalt in eine sekundäre O/W- Mikro- Emulsion überführt werden.

Die erfindungsgemäße Emulsion ist daher sowohl hinsichtlich der Wirkstoffe als auch hinsichtlich der gewünschten Phase binär differenzierbar.

Mit derartigen Zubereitungen ist daher die Haut / Haar von Säugetieren, insbesondere des Menschen kosmetisch, dermatologisch oder auch pharmazeutisch- medizinisch auf einfache Weise behandelbar, wobei die Emulsion selbst auf ebenso einfache Weise herstellbar ist.

Die erfindungsgemäße Mikro- Emulsion ist primär eine Wasser - in -Öl - Emulsion mit binärer Phasen und Wirkstoffdifferenzierbarkeit und umfasst insbesondere
a) 45 bis 90 Gew. % einer flüssigen Ölphase
b) 5 bis 40 Gew. % einer Mischung aus einem oder mehreren nicht ionischen W/O Tensiden mit einem HLB-Wert von 3-7 und einem oder mehreren nicht ionischen O/W-Tensiden mit einem HLB-Wert von 9-18 im Verhältnis 1:4 bis 1:1,2;
c) 0,01 bis 20 Gew. % eines oder mehrerer Phospholipide, Cholesterine, Cholesterin-Derivate
d) 0,00 bis 15 % Gew. % eines oder mehrerer ein- oder zweiwertigen C₁₋₈-Alkohole
   und
e.) 1 bis 10 Gew. % Wasser oder wässrige Lösungen
aufweist, wobei die Mizellen der primären Mikro- Emulsion eine Partikelgröße von 20 bis 400-nm, insbesondere 20 bis 300 nm aufweisen, und die primäre Mikro-Emulsion durch Umsetzung mit einer Wasserphase wahlweise in eine sekundäre W/O- Mikro-Emulsion oder eine O/W- Mikro - Emulsion konvertierbar ist.

Insbesondere weisen oben genannte Emulsionen in einer bevorzugten Ausführungsform 0,01 bis 15 Gew. % eines oder mehrerer Alkohole d) wie beschrieben auf.

Es ist ferner bevorzugt, wenn die Mikro- Emulsion 0 bis 30 Gew. %, insbesondere 0,01 bis 30 Gew. % eines oder mehrerer wasserlöslicher oder fettlöslicher Wirkstoffe oder Mischungen von Wasser- und Fett- löslichen Wirkstoffen aufweist.

Besonders bevorzugt sind sowohl wasserlösliche als auch fettlösliche Wirkstoffe enthalten.

Es ist auch vorteilhaft, wenn zusätzlich 0 bis 15 Gew. %, bevorzugt 0,01 bis 15 Gew. %, Zusatzstoffe vorhanden sind, die insbesondere ausgewählt sind aus Diffusions-, Penetrationverstärkern , Chelatisierungsmitteln, Elektrolyten, Oxidantien, Feuchthaltern, Bleichmitteln, Konservierungsmitteln oder deren Mischungen. Besonders bevorzugt sind hier Elektrolyte, Diffusionsverstärker, Oxidantien, chelatirende Stoffe Penetrationsförderer, Feuchthaltern oder Mischungen hiervon.

Insbesondere bevorzugt weist die erfindungsgemäße Mikro - Emulsion einen oder mehrere biologische Sauerstoffträger auf. Diese(r) ist sind insbesondere ausgewählt aus Hämoglobin, Myoglobin und Mischungen hiervon, wobei Hämoglobin bevorzugt ist. Der oder die Sauerstoffträger können in Mengen von 0,001 bis 20 Gew. %, insbesondere 0,01 bis 15 Gew. %, vor allem 0,1 bis 10 Gew. % vorliegen. Hierzu können dann in einer weiteren bevorzugten Ausführungsform die nachstehend genannten Wasser - und / oder fettlöslichen Wirkstoffe enthalten sein. Dazu können auch in einer weiteren Ausführungsform ein oder mehrere der genannten Zusatzstoffe in den angegebenen Mengen enthalten sein.

Eine weitere bevorzugte Ausführungsform stellen Mikro- Emulsionen dar, welche 50 bis 80 Gew. % einer Ölphase
5 bis 40 Gew. % einer Mischungen aus einem oder mehreren W/O- und einem oder mehreren O/W- Tensiden im oben angegebenen Verhältnis;
0,1 bis 10 Gew.% eines oder mehrerer Lecithine, Phosphatidylcholine oder Derivate oder Mischungen hiervon als Emulgator;
0,00 bis 10 Gew. %, insbesondere 0,1 bis 10 Gew. %, eines oder mehrerer ein- oder zweiwertiger C₁₋₈- Alkohole;
1 bis 10 Gew. % Wasser oder wässrige Lösungen
aufweist. Hierbei können wie geschildert Wirkstoffe der angegebenen Art in der angegebenen Menge vorhanden sein.

Die Menge an Ölen beträgt 45 bis 90, vor allem 45 bis 80, und bevorzugt 45 bis 60 Gew. %. Insbesondere besteht die Ölphase aus flüssigen Ölen , wie z.B. die Ester aus Alkancarbonsäuren. Diese sind bevorzugt ausgewählt aus Isopropylmyristat, Isopropylpalmytat, lsopropyloleat, Isooctylsterart, Isononylsteart und der gleichen mehr. Ferner bevorzugt sind Dialkylether, Fett-Alkohole mit 6 - 18 Kohlenstoffatomen oder Triglycerinester gesättigter und/oder ungesättigter Alkancarbonsäuren. Hierunter sind besonders synthetische, halbsynthetische und natürliche Öle, z. B. Olivenöl, Mandelöl, Avocadoöl, Sonnenblümenöl, Sojaöl, Erdnussöl, Rapsöl und dergleichen mehr.

Auch beliebige Abmischungen solcher Öle und Esteröle sind vorteilhaft im Sinne der vorliegende Erfindung einzusetzen.

Das Verhältnis von W/O zu O/W- Tensid oder Mischungen der Tenside beträgt 1:4 bis 1:1,2, bevorzugt 1:3 bis 1:1,2 und insbesondere 1:2 bis 1:1,3.

Die W/O- und O/W- Tenside sind bevorzugt ausgewählt aus der Gruppe der Sorbitan Ether, der Ethoxylierten Sorbitan Derivate. Ferner können die Tenside vorteilhaft gewählt werden aus der Gruppe Ethoxylierte Fettalkohole mit 8-18 Kohlenstoffatomen in geraden Ketten, der Glyceryl- Ether / Ester von gesättigten und ungesättigten Fettsäuren, ethoxylierte Glyceryl Ester, bevorzugt Di- und Triglyceride, der ethoxylierten Alkylether, oder der Fettalkohol- (C16-C18)-Glukoside oder geeigneten Mischungen der jeweiligen Tenside.

Die Menge an nichtionischen Tensiden (eine oder mehrere Verbindungen) in den Zubereitungen beträgt insbesondere 5 bis 35 Gew.%, bezogen auf das Gesamtgewicht der Zubereitung.

Die Emulgator-Komponente c) ist insbesondere ausgewählt aus Lecithinen oder Phosphatidylcholinen oder Derivaten oder Mischungen hiervon, wie Lecithin aus Pflanzen (Soja, Raps, Baumwollesamen) und Eigelb; Phosphatidylcholin aus Soja und Eigelb; Gemische aus Phosphatidylcholin und Lecithin in unterschiedlichen Verhältnissen wie NAT-Produkte, Phosohatidylethanoiamin; Phosphatidylserin; Phospatidylinosit aus Soja, Raps, Baumwollesamen; hydroxyliertes Lecithin. Die Menge an diesem oder diesen Emulgator(en) in den Zusammensetzungen beträgt 0,01 bis 20, bevorzugt 15 Gew. %, vor allem 0,1 bis 10 Gew. %, bevorzugt 0,5- 5 Gew.%, insbesondere 1- 5 Gew. % bezogen auf das Gesamtgewicht der Zusammensetzung.

Der oder die Alkohole sind bevorzugt ausgewählt aus einwertigen C₁₋₈- Alkoholen wie Ethanol, Propanol, Isopropanol; oder aus zweiwertigen Alkoholen wie Glykole z.B. Propylenglykol, 1,2-Oktandiol; 1,2-Hexandiol.

Die Menge an Alkohol(en) in den Zusammensetzungen beträgt 0,0bis 15, vor allem 0,01 bis 15 und insbesondere 0,1 bis 15 Gew. %, vorzugsweise 1 bis 15 Gew. %, besonders bevorzugt 5- 15 Gew. %, insbesondere 5 bis 10 Gew. % bezogen auf das Gesamtgewicht der Zusammensetzung.

Bevorzugt ausgewählte wasserlösliche Wirkstoffe sind Aminosäuren, Peptide, Protein-Hydrolisate, Proteine, Saccharide, Oligosaccharide, Polysaccharide und Derivate hiervon, Hormone und Hormon- ähnliche Stoffe, Antioxidantien, Vitamine und Provitamine, AHA-Säuren, Feuchthalter wie NMF, Oxidationsmittel, pflanzliche Extrakten, Flavonoiden und pflanzliche Polyphenole oder Mischungen hiervon.

Bevorzugt ausgewählte fettlösliche Wirkstoffe sind Antioxidantien, Vitamine, Provitäminen, ungesättigte Fettsäuren, Ceramide oder Mischungen hiervon. Ggf. können auch Prostaglandine, insbesondere aber dermatologisch wirksame Mittel, ausgewählt aus Hormonen und Hormon- ähnlichen Stoffen, Antymikotika, Keratinolytika, Keratinoplastika, Narbenmittel, Gerbstoffen, Teeren, Azelaïnsäure, Photocumarinen oder Mischungen hiervon.

In einer besonders bevorzugten Ausführungsform weist die erfindungsgemäße Mikro- Emulsion Proteine, ausgewählt aus nativem, modifizierten und / oder unmodifiziertem Hämoglobin, Myoglobin oder Mischungen- hiervon, insbesondere nicht modifiziert, in einer Gesamtmenge von 0,001 bis 20 Gew.% , insbesondere 0,01 bis 20 Gew. % oder auch 0,1 bis 20 Gew. %., insbesondere 1 bis 15 Gew. %, vor allem 1 bis 10 Gew. % auf.

Hierbei ist es bevorzugt, wenn zusätzlich weiterhin Antioxidantien, Proteinstabilisatoren, Mono-, Oligo- und Polysaccharide, insbesondere Glukose, Kollagen, Feuchthalter, Aminosäuren oder Mischungen hiervon als Zusatzstoffe enthalten sind. Insbesondere sind, vor allem auch bei Hämoglobin / Myoglobinhaltigen Produkten, weiterhin Antioxidantien, Glutathion, Superoxid-Dismitase, Melatonin, Flavonoide, Aminosäuren, ggf. weiterhin sowie Kollagen, Glukose , Aminosäuren und Feuchthalter, oder Mischungen hiervon vorhanden sind.

Eine ganz besonders bevorzugte erfindungsgemäße Mikro- Emulsion umfasst als Wirkstoffe unmodifiziertes Hämoglobin, Myoglobin oder Mischungen hiervon in einer Menge von 1 bis 10 Gew. % insgesamt, sowie 1 bis 5 Gew. % Glukose, 0,01 bis 5 Gew. % der für Menschen natürlichen Aminosäuren.

Hierbei werden vor allem Emulsionen bevorzugt, die neben der Wasserphase 50 bis 90 Gew. % einer flüssigen Ölphase mit Ölen ausgewählt aus lsopropylmyristat, lsopropylpalmytat, Isopropyloleat, Isooctylsterart, Isononylstearat aufweisen. Ferner bevorzugt sind Dialkylether, Fett-Alkohole mit 6 - 18 Kohlenstoffatomen oder Triglycerinester gesätigter und/oder ungesättigter Alkancarbonsäuren. Hierunter sind besonders synthetische, halbsynthetische und natürliche Öle, z. B. Olivenöl, Mandelöl, Avocadoöl, Sonnenblümenöl, Sojaöl, Erdnussöl, Rapsöl sowie 0,1 bis 40 Gew %, vor allem 0,1 bis 30 oder auch 0,1 bis 20 Gew. % an Tensidmischung, ausgewählt aus der Gruppe der Sorbitan Ether, der Ethoxylierten Sorbitan Derivate geeignet.

Ferner können die Tenside vorteilhaft gewählt werden aus der Gruppe ethoxylierte Fettalkohole mit 8-18 Kohlenstoffatomen in geraden Ketten, der Glyceryl- Ether von gesättigten und ungesättigten Fettsäuren, ethoxylierte Glyceryl Ester, der ethoxylierten Alkylether, oder der Fettalkohol- (C16-C18)-Glukoside oder geeignete Mischungen der jeweiligen Tenside mit HLB-Wert < 8 als W/O- Tenside und als O/W- Tenside solche aus der Gruppe der Sorbitan Ether, der Ethoxylierten Sorbitan Derivate. Ferner können diese Tenside vorteilhaft gewählt werden aus der Gruppe Ethoxylierte Fettalkohole mit 8-18 Kohlenstoffatomen in geraden Ketten, der Glyceryl- Ether von gesättigten und ungesättigten Fettsäuren, ethoxylierte Glyceryl Ester, der ethoxylierten Alkylether, oder der Fettalkohol- (C16-C18)-Glukoside oder geeigneten Mischungen der jeweiligen Tenside mit HLB-Wert >10, insbesondere zusammen mit 0,1 bis 8 Gew. % Phosphatidylcholin oder Phosphatidylcholinhaltigen Produkten wie z.B. NAT-8539 (Rhone-Poulenc) sowie 0,1 bis 5 Gew. % Ethanol, Isopropanol , 1,2-Octandiol oder Mischungen hiervon.

Es kann auch von Vorteil sein, wenn die o.g. Mikro- Emulsionen zusätzlich Wasserund/ oder Fettlösliche Vitamine/ Provitamine in einer Menge von 0,01-1,0 Gew. % enthält.

In einer weiteren bevorzugten Ausführungsform weist die erfindungsgemäße Emulsion als Wirkstoff oder als zusätzlichen Wirkstoff Pflanzenextrakte in einer Menge von 0,1 Gew. % bis 5, insbesondere bis 3,0 Gew. %, 0,1 bis 5,0 Gew. % etherische Öle, 0,1 bis 10 Gew. % AHA-Säuren, 0,01 bis 0,3 Gew. % Hormone oder Hormonähnliche Stoffe, 0,1 bis 5 Gew. % essentielle Fettsäuren, Ceramide (0,1 bis 5 Gew. %) oder Mischungen hiervon auf.

Wie erwähnt kann die erfindungsgemäße Mikro- Emulsion topisch appliziert werden oder eine Mischung hiervon mit 1 bis 90, bevorzugt 10 bis 90 Gew. % einer Wasserphase wird bereitet, wobei die Zubereitung dann 1 bis 50 oder auch 5 bis 50, insbesondere 1 bis 40 Gew. % der Ölphase aufweist und eine W/O oder eine O/W-Mikro - Emulsion darstellt.

Diese primären Mikro-Emulsionen können vor allem von 1 bis 30% Wasser oder wässrige Lösungen aufnehmen und dadurch bildet sich eine sekundäre W/O - Mikro-Emulsion mit in Wassertröpfchen eingeschlossenen Stoffen. Die Tröpfchen haben einen Durchmesser zwischen 50 und 400 nm.

Durch Verdünnung mit Wasser oder wässrigen Lösungen können die primären Mikro-Emulsionen sich in O/W- Mikro-Emulsionen konvertieren, die Öl-Tropfen (1-40%) mit einem Durchmesser von 40 bis 300-nm-in-eine kontinuierliche-wässrige Phase (60-95%) haben.

Eine derartige Mischung kann insbesondere 26 bis 50 Gew. % Ölphase aufweisen und eine sekundäre W/O- Mikro-Emulsion darstellen oder 5 bis 25 Gew. % Ölphase haben und dann eine sekundäre O/W- Mikro- Emulsion darstellen.

Die erfindungsgemäßen Mikro- Emulsionen werden hergestellt, indem man die Ölphase, enthaltend das oder die Tenside, das oder die Emulgatoren der Komponente c), enthaltend den oder die Alkohole und ggf. darin vorhandene fettlösliche Wirkstoffe, mit einer Wasserphase, und ggf. den oder die wasserlöslichen Wirkstoffe, bei Temperaturen von 10 bis 30 °C miteinander vermischt, und die so erhaltene primäre W/O- Mikro-Emulsion ggf. mit einer Wasserphase, die ggf. weitere wasserlösliche Wirkstoffe aufweisen kann, in eine sekundäre W/O- oder eine sekundäre 0/W/- Mikro-Emulsion überführt.

Die erfindungsgemäßen Mikro- Emulsionen eignen sich insbesondere als kosmetische, dermatologische, pharmazeutische Zubereitungen zur topischen Behandlung einer allergisch, bakteriell, immunologisch, durch äußere Einflüsse beeinträchtigten, irritierten oder beschädigten und degenerierten Haut oder zur Pflege oder Behandlung von Haaren, wie z.B. zur Reinigung oder Konditionierung, letzteres z.B. bei trockenem, angegriffenem schwer kämmbarem Haar.

Ganz besonders können sie zur Behandlung von altersbedingt irritierter oder veränderter Haut eingesetzt werden. Hierzu sind vor allem die Zubereitungen, enthaltend Hämoglobin, Myoglobin oder Mischungen hiervon, vorteilhafter weise mit den oben beschriebenen weiteren Wirkstoffen, Zusatzstoffen, wie insbesondere Wasserstoffperoxid, geeignet.

Besonders ist auch die Behandlung von Neurodermitis, Akne und Psoriasis möglich. Auch kann das erfindungsgemäße Mittel gegen Entzündungen, auch bei Einsatz der Sauerstoffträger, eingesetzt werden.

Es hat sich gezeigt, dass mittels der erfindungsgemäßen Emulsion selbst die genannten Wirksubstanzen bis zu den vitalen Zellen des Stratum germinativum penetrieren, insbesondere die erwähnten biologischen Sauerstoffträger, Glukose, und ggf. die weiterhin vorhandenen Wirk- und / oder Zusatzstoffe wie Feuchthalter, Vitamine, essentielle Fettsäuren und Lipide, Spurenelemente, Antioxidantien , Aminosäuren, - weiterhin auch Peptide, Monosaccharide, Oligosaccharide und Polysaccharide, Oligonukleotide, Hilfsstoffe.

Die erfindungsgemäße primäre und sekundäre W/O-Mikro- Emulsion weist somit in einer besonders bevorzugten Ausführungsform eine kontinuierliche Ölphase auf, die Tröpfchen der diskontinuierlichen Wasserphase enthält, die im Wesentlichen folgende Bestandteile aufweisen kann:
einen oder mehrere biologische Sauerstoffträger, ggf. in Verbindung mit einem oder mehreren Protein-Stabilisatoren; ein oder mehrere Antioxidantien;
ein oder mehrere Vitamine und Provitamine; ein oder mehrere Mono-, Oligo und Polysaccharide und deren Derivate; ein oder mehrere Aminosäure, Peptide und Proteinhydrolysate; ein oder mehrere Proteine und Proteinderivate; ein oder mehrere Hormone und hormonähnliche Substanzen; ein oder mehrere pflanzliche Extrakte;
ein oder mehrere Diffusionsverstärker; einen oder mehrere Penetrationsförderer; ein oder mehrere anorganische Salze; ein oder mehrere Chelatbildner; ein oder mehrere oxidierende Stoffe (H₂O₂, Hydrochinon); ein oder mehrere chemische Sauerstoffträger; ein oder mehrere wasserlösliche Wirkstoffe (Pharmazeutika, Dermatika).

Die Ölphase weist insbesondere die Tenside, den oder die Emulgatoran der Komponente c) und Alkohole, Antioxidantien, Vitamine und Provitamine, essentielle Fettsäuren, Ceramide, Prostaglandine, etherische Öle, lipophile Wirkstoffe aus der Gruppe der Pharmaka, Dermatika auf.

Dabei sind die einzelnen Inhaltsstoffe in den oben angegebenen Mengen, insbesondere den bevorzugten Anteilen vorhanden.

Erstaunlicherweise wurde gefunden, dass Hämoglobin oder Myoglobin eingebunden in die erfindungsgemäße Mikro-Emulsion schnell und tief in das Stratum corneum eindringen kann, und sich dort homogen verteilt (vgl. Anwendungsbeispiel). Hiermit wird eine für in die Hornschicht eingebrachtes Hämoglobin erleichterte Diffusion von Sauerstoff bewirkt.

Für die optimale Wirkung der erleichterten Diffusion müssen Sauerstoff-Affinität und Sauerstoff-Kooperativität des Sauerstoffbinders (Hämoglobin, Myoglobin) günstig eingestellt werden Ein Maß für die erstere ist der Halbsättigungsdruck(P₅₀), für letztere der HILLsche Index. Die Kooperativität muss möglichst groß sein, für Vollblut ist ihr Wert etwa 2,6.

Die Affinität dagegen muss einerseits so groß sein (d.h. der P₅₀ wert so klein), dass der Sauerstoff aus der Luft noch gut aufgenommen ist, aber andererseits muss die Affinität so gering sein, dass der aufgenommene Sauerstoff auch leicht wieder an die Zellen des Hautepithels abgegeben werden kann. Hämoglobin wirkt erfindungsgemäß auf zweierlei Weise anti-oxidativ, nämlich, wie erläutert, durch Herabsetzen der Sauerstoff-Spannung und durch seine Katalase-Wirkung. Eine dritte Wirkung ist Entzündungshemmung und eine vierte der Lichtschutz.

Hinsichtlich der biologischen Sauerstoffträger ist es vor allem bei Hämoglobin von Vorteil, die Affinität chemisch zu modifizieren, dies kann aber auch durch nicht kovalent gebundene Effektoren geschehen, die der Zubereitung beigemischt werden. Eine chemische Modifizierung kann z. B. mit Pyridoxalphosphat erfolgen.

Eine kovalente Modifizierung kann z.B. mit 2,3.Diphosphoglycerat oder künstlichen Effektoren wie Inositolhexaphosphat oder Mellitsäure, in 1-3facher, insbesondere etwa äquivalenter Menge, bezogen auf Hämoglobin oder Hämoglobin/Myoglobin, erfolgen.

Gegebenenfalls kann eine weitere Stabilisation des biologischen Sauerstoffträgers bezüglich der Funktionsstruktur der Proteine erfolgen, denn die in der Emulsion vorhandenen Tenside könnten auch die Funktion der Sauerstoffbindung des Hämoglobins beeinflussen.

Besonders bevorzugt ist ein mit Kohlenmonoxid (CO) stabilisiertes Hämoglobin vom Menschen oder Rind, vorzugsweise jedoch Schweinehämoglobin. Die Herstellung eines solchen stabilisierten Hämoglobins ist in der DE 1 970 103.7 beschrieben (entsprechend US-Patent Nr. 5,985,332). Demnach kann Hämoglobin/Myoglobin durch Aquilibrierung mit CO vollständig in Carboxyhämoglobin/Myoglobin überführt werden, welches lagerstabil ist und vor der weiteren Anwendung nicht deligandisiert werden muss. Die Karbonylierung ist auch mit modifiziertem Hämoglobin möglich.

Die Aktivierung des Sauerstoffträgers kann dann durch lokale Begasung der Haut mit Sauerstoff, auf die die Emulsion aufgetragen worden ist.

Das-Hämoglobin kann, wie erwähnt, in einer Mischung mit Myoglobin, insbesondere letzteres in Mengen von 0,1 bis 50 %, bezogen auf die Hämoglobin-Menge, vorliegen. Bevorzugt wird Myoglobin mit Hämoglobin in Mengen von 50 bis 70% Hämoglobin und 50-30% Myoglobin, insbesondere 75 bis 90% Hämoglobin und 25 bis 10% Myoglobin eingesetzt. Die %-Angaben sind hierbei Masseanteile.

Als Hämoglobin kann insbesondere Humanhämoglobin, Schweinehämoglobin, welches bevorzugt ist, oder Rinderhämoglobin eingesetzt werden. Die Art des Myoglobins ist ebenfalls wählbar, es kann von verschiedenen Tierarten gewonnen werden, wie z. B. vom Hund, vom Schaf, vom Pferd oder vom Wal.

Besonders bevorzugt wird nicht modifiziertes natives Hämoglobin und/ oder Myoglobin eingesetzt, welches insbesondere bevorzugt durch Karbonylierung gegen Oxidation geschützt sein kann, wobei die Sauerstoffträgerlösung einen nicht chemisch reaktiven Effektor, wie erwähnt insbesondere 2,3-Diphosphoglycerat aufweist, in einer 1 bis 3 fachen, vorzugsweise äquivalenten Menge bezüglich des Hämoglobins/Hämoglobins/Myoglobins aufwies. Ferner kann zusätzlich oder alternativ auch mit Pyridoxal-Effektoren chemisch modifiziertes Hämoglobin eingesetzt werden. Dazu wird Hämoglobin mit den entsprechenden genannten Effektoren umgesetzt, gegebenenfalls karbonyliert.

Ganz bevorzugt wird erfindungsgemäß desoxygeniertes, gegebenenfalls karbonyliertes nicht modifiziertes Human- oder insbesondere Schweinehämoglobin und entsprechendes desoxygeniertes, nicht modifiziertes Myoglobin vom Hund, oder Schaf, Pferd eingesetzt.

Als geeignete Elektrolye zusammen mit den Sauerstoffträger(n) können NaCl, KCI und NaHC03, insbesondere in physiologischen Mengen vorliegen (in mM): NaCl 125; KCI 4,5; NaHCO₃ 20) vorliegen.

Alternativ kann als Modifikation Hämoglobin oder Myoglobin auch zur Stabilisierung mit Polyalkylenoxiden verknüpft sein, wie in den Patenten US B 4 179 337, US B 5 478 805, US B 5 386 014, US B 5 312 808 oder EP 0 206 448, EP 067 029 beschrieben. Ferner kann der Sauerstoffträger auch polymerisiert oder polymerisiert und pegyliert, mit Effektoren umgesetzt und oder karbonyliert sein wie in DE A 100 31 740 (WO 02/00230), DE A 100 31 744, DE A 100 31 742 beschrieben.

Überraschenderweise wurde gefunden, dass man eine Hämoglobin oder Myoglobin enthaltende Mikro-Emulsion unmittelbar vor der Anwendung herstellen kann.

Erstaunlicherweise können hierbei verschiedene Haut-Tönungen erzielen werden (vgl. Anwendungsbeispiel).

Für die kosmetische und dermatologische Behandlung normaler, gealterter und degenerierter Haut können primäre Mikro-Emulsionen gemäß vorliegender Erfindung in Form fein-dispergierter Wassertropfen (0,1 - 40 %) mit einem Durchmesser von 30 bis 400 nm in einer kontinuierlichen, Tenside- und Alkohol/Emulgator enthaltenden (1-40%), Öl-Phase (30-70%) hergestellt werden.

Diese primären Mikro-Emulsionen können von 1 bis 30% Wasser oder wässrigen Lösungen aufnehmen und dadurch bildet sich eine sekundäre W/O - Mikro-Emulsion mit in Wassertröpfchen eingeschlossenen Stoffen. Die Tröpfchen haben einen Durchmesser zwischen 50 und 400 nm.

Durch Verdünnung mit Wasser oder wässrigen Lösungen können die primären Mikro-Emulsionen sich in O/W- Mikro-Emulsionen konvertieren, die Öl-Tropfen (1-40%) mit einem Durchmesser von 40 bis 300 nm in einer kontinuierlichen wässrigen Phase (60-95%) haben.

Sowohl die primären als auch die sekundären Emulsionen können sehr schnell und tief in das Stratum corneum der Haut eindringen, wobei W/O- Mikro-Emulsionen als flüssig bis Flüssiggele oder Gele vorliegen und die O/W- Mikro-Emulsionen flüssig und versprühbar vorliegen.

Dabei liegt keine systemische Wirkung vor, wie anhand eines Insulin- Tests ermittelt werden konnte. An 2 Versuchspersonen wurde eine Mikro- Emulsion gemäß folgendem Beispiel 1, Tabelle 1, Produkt 1 , enthaltend Insulin in üblichen Mengen, appliziert. Eine Bestimmung des Blutglukosespiegels nach 3 Stunden ergab keinen Abfall.

Im folgenden werden die einzelnen Bestandteile der erfindungsgemäßen Mikro-Emulsion näher beschrieben:

### 1. Öle

Die Ölphasen der erfindungsgemäßen Mikro-Emulsionen werden vorteilhaft ausgewählt aus folgenden Substanzen:
a.) Ester aus C₉₋₁₈ Alkancarbonsäuren und C₃₋₃₀- Alkoholen wie insbesondere lsopropylmiristat, lsopropylpalmitat, lsopropylstearat, lsopropyloleat, n-Butylstearat, n-Hexyllaurat, n-Decyloleat, lsooctylstearat, Isononylstearat, Isononylisonanoat, 2-Ethylhexylpalmitat, 2-Ethylhexyllaurat, 2-Hexyldecylstearat, 2-Octyldodecylpalmitat, Ethyloleat, Oleyloleat, Oleylecurat, Erucyloleat sowie synthetische, halbsynthetische und natürliche Gemische solcher Ester. Bevorzugt wird die Ölphase gewählt aus pharmazeutisch verträglichen Ölen wie Isopropylmiristat, Ethyloleat, Isopropylpalmitat,lsopropyloleat, Oleyloleat, Isooctylstearat.
b.) Gesättigte, ungesättigte langkettige Fettsäuren tierischer und pflanzlicher Herkunft, insbesondere Olein-, Palmytin- oder Ölsäure, oder Folsäure und deren Derivate, Extrakte oder andere Produkte pflanzlicher und tierischer Herkunft, z.B. Nachtkerzenöl, Borretschöl oder Johannisbeerkernöl, Besonders sind auch Nachtkerzenöl, Borretschöl, γ- -Linolensäure;.
c.) Dialkylether, der Gruppe der Alkohole, sowie der Fettsäuretriglyceride, insbesondere Triglycerinester gesättigter und/oder ungesättigter Alkancarbonsäuren einer Kettenlänge von 8 bis 24, insbesondere 12 bis 18 C-Atomen. Besonders bevorzugt sind synthetische, halbsynthetische und natürliche Öle, z.B. Olivenöl, Mandelöl, Avocadoöl, Sonnenblumenöl, Sojaöl, Erdnussöl, Rapsöl, Palmöl, Kokosöl, Palmkernöl und dergleichen mehr. Ferner bevorzugt sind die pharmazeutisch verträglichen Öle 2-Ethylhexylisostearat, Octyldodecanol, Isotridecylisonanoat, Isoeicosan, 2-Ethylhexylcocoat, Capryl-Caprinsäure-triglycerid, Dicaprylylether.
d.) Schwerflüchtige Kohlenwasserstoffe, insbesondere Paraffinöl, Squalen und Squalan.
e.) Fettalkohole mit 6 -18 Kohlenstoffatomen in geraden Ketten wie z. B. Lauryl-, Palmitin-, Myristin-, Arachidon, Linolen- und Linolalkohol.

Besonders bevorzugte Öle sind neben den bereits erwähnten auch Isopropylmiristat, Isopropylpalmitat, Isopropylstearat, Isopropyloleat, Olivenöl, Mandelöl, Avocadoöl, Sonnenblumenöl, Sojaöl, Erdnussöl, Rapsöl, Capryl-Caprinsäure-triglycerid, Dicaprylylether, Squalan oder Mischungen hiervon.

Darüber hinaus sind auch Mischungen einzelner Öle sowohl aus einzelnen Gruppen wie auch aus verschiedenen Gruppen möglich.

### 2. O/W und W/O- Tenside

Die Tenside sind ausgewählt aus nichtionischen Substanzen. Aus dieser Gruppe gibt es sowohl O/W- als auch W/O- Tenside. Erstere sind u. a. gekennzeichnet durch einen HLB-Wert ≥ 8, letztere weisen üblicherweise einen HLB- Wert von ≤ 8 auf. Bevorzugt werden Tenside mit einem HLB- Wert von 2 bis 6 zusammen mit solchen mit einem HLB- Wert von 12 bis 20 eingesetzt.
a.) Sorbitan Derivate, insbesondere Sorbitanmonolaurat und Sorbitantrioleat:
b.) Ethoxylierte Sorbitan Derivate, wie vor allem Polyethylenglycol(20)-Sorbitanmonolaurat, Poliethylenglycol(20) sorbitanmonostearat, Polyethylenglykol(20)sorbitanmonooleat.
c.) Glyceryl Ether von gesättigten und ungesättigten Fettsäuren wie z. B. mono-, di-, tri- Glycerin und Polyglyceryl- Derivate, einschließend Polyglyceryl Diisostearate, Polyglyceryl-2-Oleylether, Polyglyceryl-6-distarat, Polyglyceryl-4-oleylether.
d.) Ethoxylierte Glyceryl Ester wie ethoxylierte Triglyceride, z. B. Polyethylenglycol(20)- Glyceryltristearat ; Unter den Glyceriden sind Di -und Trigyceride/ Derivate hiervon bevorzugt.
f.) Ethoxylierten Alkylether, insbesondere Polyethylenglykoldodecylether (Brij30), Polyethylenglykol-hexadecylether (Brij52).
g.) Fettalkohol- (C16-C18)-Glukoside wie Sucrose-Stearat, Sucrose-palmitat, Plantacare 1200 UP und Plantacare 2000 UP
h.) Ethoxylierte Fettalkohole mit 8-18 Kohlenstoffatomen in geraden Ketten, insbesondere (Polyethylenglycol(2) stearylether (Steareth-2),von Stearech13 bis Steareth-20, von Oleth-3 bis Oleth-15., von Cetech13 bis Cetech-20, von Ceteareth 12 bis Ceteareth-30 (INCI).

Die Menge der nichtionischen Tenside (eine oder Mehrere Verbindungen) in den Zubereitungen beträgt insbesondere 10- 40 Gew. %, bezogen auf das Gesamtgewicht der Zubereitung.

### 3. Emulgatoren

a.) Phospholipide, insbesondere Lecithin aus Pflanzen (Soja, Raps, Baumwollesamen) und Eigelb; Phosphatidylcholin aus Soja und Eigelb oder Mischungen hiervon wie NAT 8539); Phosphatidylethanolamin; Phosphatidylserin, Phospatidylinosit aus Soja, Raps, Baumwollsamen, hydroxyliertes Lecithin, Gemische aus Phosphatidylcholin und Lecithin in variablen Anteilen wie z. B. NAT 8539 oder ähnliche. Insbesondere bevorzugt sind Lecithin aus Soja und Eigelb, z.B. unter Handelsbezeichnungen Epikuron 135, Epikuron 170, Epikuron 200, Epikuron 200 SH (Lukas Meyer), Phospholipon 25, NAT-8539 (Nattermann).
b.) Cholesterin und Cholesterin-Derivate wie beispielsweise ethoxyliertes Cholesterin wie Polyethylenglycol(10)- Sojasterol verwendet werden.

### 4. Alkohole

a.) einwertige C_{1-8'}- Alkohole wie Ethanol, Propanol, Isopropanol, Butanol;
b.) Glykole wie Propylenglykol, 1,2-Oktandiol, 1,2- Hexandiol; Insbesondere bevorzugt sind Ethanol, Propanol, 1,2 Octandiol , Propylenglykol.

### 5. Wirkstoffe

### 1. Fettlösliche Wirkstoffe

a.) Fettlösliche Vitamine wie Vitamin A und den Derivaten, Vitamin C und Derivaten, Vitamin E und Derivate. Insbesondere bevorzugt sind Tocopherol-Acetat, Ascorbinsäure-Palmitat;
b.) Antioxidantien wie Carotinoide, Carotine (z.B. α- und β-Carotine und deren Derivate), α-Tocopherol und Derivaten, Ascorbinsäure Palmitat.
c.) Ätherische Öle, insbesondere Terpene (Mono-, Sesquiterpene, Diterpene): Citrusöl, Pinie, Kamille; Alkohole (Monoterpenole, Sesquiterpenole, Diterpenole): Ravensara, Ysop, Niaouli; Aldehyde: Melisse, Eucalyptus; Ketone (Monoterpenketone, Sesqui- und Diterpenketone): Scharfgabe, Thuja, sowie Ester (Monoterpenester): Lavendelöl, Ylang Ylang; Phenole: Thymian; Phenylether: Anis, Nelke; Oxide: (Cineol); Lactone: Patchouli, Weihrauch und Kumarine.
   Ganz besonders bevorzugt sind Nelkenöl, Thymianöl, Minzöl, Citrusöl, Pinie, Lavendelöl, Ylang Ylang, Kamille, Ravensara, Ysop, Niaouli, Anis, Patchouli, Weihrauch, Scharfgabe, Thuja, Birkenöl, Melisse, Eucalyptus.
   Ferner werden die etherischen Ölen bevorzugt ausgewählt aus Citrusöl, Pinie, Kamille, Ravensara, Ysop, Niaouli, Melisse, Eucalyptus, Schafgarbe, Thuja, Lavendelöl, Ylang Ylang, Teebaumöl.
   Die Menge der ätherischen Öle (eine oder mehrere Verbindungen) in den Zubereitungen beträgt vorzugsweise 0,1 bis 10 Gew. %, besonders bevorzugt 0,5- 5. Gew. %, insbesondere 1- 5 Gew.% bezogen auf das Gesamtgewicht der Zubereitung.
d.) Essentielle Fettsäuren, insbesondere Linol- und y - Linolensäuren, oder essentielle fettsäurenhaltige Öle. Besonders bevorzugt γ-Linolensäure, Borretschöl, Nachtkerzenöl.
e.) Ceramide wie Ceramid I, Ceramid III.

### 2. Wasserlösliche Wirkstoffe

a) Antioxidanten wie Hydroxy- und Dihydroxybenzoate, Hippurate, Salicylate, Cystein und Derivate hiervon, Glutathion, Vitamin C und dessen Derivate(z.B. Mg - Ascorbylphosphat, Ascorbylacetat), Vitamin H, Superoxid-Dismutase, Katalase; Aminosäuren (z.B. Glycin, Histidin, Thryoin) und deren Derivate, Imidazole, Thiolen (Glutathion, Thioredoxyn, Cystein, Cystamin und deren Derivate), Flavonoide, Melatonin, Vitamin E und dessen Derivate.
   Insbesondere bevorzugt sind Ascorbylacetat, Superoxid-Dismutase, Cystein und Glutathion.
b.) Vitamine und Provitamine wie z. B. Vitamin B-Komplex, Vitamin C und Derivate, Vitamin H und Derivate, Biotin, Pantothensäure, Pantenol.
c.) Saccharide und Oligoasaccharide wie z.B. Glukose, Fructose, Mannose, Mannitol, Inosit, N-Acetyl-D-Glukosamin, D-Glukosamin, Chito-Oligosaccharide, Raffinose, Trehalose.
   Besonders bevorzugt sind Saccharide und Oligoasaccharide, insbesondere Glukose, D-Glukosamin, N-Acetyl-D-Glukosamin, Chito-Oligosaccharide; Trehalose.
d.) Polysaccharide wie Chitosan, Hyaluronsäure, Heparin, Dextran, Zellulose Ester, Alginsäure.
   Besonders bevorzugt sind hier Chitosan, Hyaluronsäure.
e.) Proteine und Proteinderivate wie z. B. Hämoglobin, Myoglobin, Kollagen, Fibrin, Elastin. Besonders bevorzugt ist hier neben den Sauerstoffträgern Kollagen.
f.) Hormone und hormonähnliche Substanzen wie z. B. Hydrocortison und dessen Derivate, Melatonin, Glycyrrhitinsäure und deren Derivate, sowie andere pflanzliche Hormone. Insbesondere bevorzugt sind hier Melatonin, Glycyn-hitinsäure und Derivate.
g.) Pflanzliche Extrakte (Extrakte selbst oder andere Produkte pflanzlicher und tierischer Herkunft) werden vorteilhaft ausgewählt aus Meristeme-Extrakt, Aloe Vera, Echinacea, Hammamelis Extrakt, Spargelextrakt, Niembaum, Rosskastanie, , Arnika, Ringelblume.
   Besonders bevorzugt sind Meristeme-Extrakt, Aloe Vera, Echinacea, Efeu, Brennessel, Kamille, Schachtelhalm.
h.) Aminosäuren. Peptide und Protein-Hydrolysate, wie alle für Säugetiere, insbesondere Menschen geeignete natürliche Aminosäuren, Protein-Hydrolysate, z.B. Seideprotein Hydrolysat, Hefehydrolysat, Weizen-Proteinhydrolysate. Hier sind besonders bevorzugt die natürlichen Aminosäuren, Peptide und Proteinhydrolysate wie Seidenproteinhydrolysat, Hefehydrolysat.
i.) Chemische Sauerstoffträger, wie organische und anorganische Peroxyde, z.B. Wasserstoffperoxid, Benzoylperoxid. Bevorzugt ist Wasserstoffperoxid.
k.) Feuchthalte-Substanzen wie die sog. NMF(aktoren), insbesondere Glycerin, Ectoine, Sorbitol, Pyrrolidoncarbonsäure, Harnstoff, Allantoin, Glukosamin, Trehalose, Chito-Oligosaccharide, Carbonsäure, Hydroxycarbonsäure und Dicarbonsäure, als auch Polysaccharide - Hyaluronsäure,Chitosan, Aloe Vera-Extrakt. Bevorzugt werden Glycerin, Harnstoff, Sorbitol, Allantoin, Pyrrolidoncarbonsäure , Milchsäure, Hyaluronsäure, Chitosan, Extrakt Aloe Vera, Chitooligosaccharide gewählt.

Die Menge solcher Wirkstoffe(eine oder mehrere Verbindungen) in der Zubereitung beträgt vorzugsweise 0,01 bis 15 Gew. %, besonders bevorzugt 0,5-10 Gew. %, insbesondere 1-5 Gew. % auf das Gesamtgewicht der Zusammensetzung bezogen.

### 6. Zusatzstoffe

Die erfindungsgemäßen Mikro- Emulsionen können auch einen oder mehrere der folgenden Zusatzstoffe aufweisen. Dabei hat sich gezeigt, dass z. B. Elektrolyten keinen Einfluß auf die Emulsion ausüben wie im Stand der Technik berichtet, nämlich die Hydrophole-Lipophile-Ballance der Tenside nicht beeinflussen.
a.) Elektrolyte, insbesondere eines oder mehrere Salze mit folgenden Anionen: Chlorid, Sulfat, Karbonat, Phosphat. Auch auf organischen Anionen basierende Elektrolyte können vorteilhaft verwendet werden, beispielsweise Laktate, Acetate, Benzoate, Salizilate, Propinate, Tartrate, Citrate und andere mehr.
   Als Kationen der Salze werden bevorzugt Ammonium,- Alkylammonium,-AlkaliMetall,- Erdalkalimetall,- Magnesium,- Eisen,- und Zinkionen verwendet Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten in Mengen von 0,1 bis 2,0%.
b.) Chelatierende Stoffe (Ethylendiamintetraessigsäure und deren Salze, Deferoxamin, Histidin). Bevorzugt ist hier Ethylendiamintetraessigsäure
c.) Chemische und natürliche Bleichmittel, z.B. Hydrochinone, Kojaksäure, Arbutin, Azelaïnsäure, Zitronen- und Gurkensaft. Hierbei sind besonders, Arbutin, Azelainsäure oder auch Hydrochinon bevorzugt..
d.) Chemische und natürliche Bräunungsmittel wie Walnussschalenextrakt, Alloxan, 1,3-Dihydroxypropan-2-on.
e.) Konservierungsstoffe wie Salizilate, Benzoate, Parabene , wobei Salizylsäure und Phenylethanol besonders bevorzugt sind.
f.) Diffusionsverstärker wie Menthol und andere aus der Gruppe, Pinen, Thymol, Campher, Koffein, Diethylenglykol-Ether, z.B. Diethylenglykol-monoethylether, Diethylenglykol, Cineol, Menthol, Propylenglykol, Butylenglykol, Polyethylenglykol von 4 bis 250 Ethylenglykol-Gruppen, Diethylenglykol Ester, z.B. Diethylenglykolmonoethylester, Oleinsäure, , Salizylsäure, α-Hydroxysäuren. Vorteilhaft werden die Diffusionsverstärker gewählt aus der Gruppe Menthol und Diethylenglykolmonoethylether.
g.) Penetrationsverstärker wie Harnstoff, Ethanol, Dimethylsilfoxid ,Cystein. Die Zusatzstoffe liegen je nach chemischer Beschaffenheit zunächst in der Wasser - oder in der Ölphase vor.
   Die Menge an Zusatzstoffen beträgt bevorzugt 0,1 Gew. % bis 10 Gew. %

Es ist gegebenenfalls möglich und vorteilhaft, die erfindungsgemäßen Zubereitungen als Grundlage für pharmazeutische Formulierungen zu verwenden. Die Übergänge zwischen reinen Kosmetika und reinem Pharmaka sind dabei fließend. Als pharmazeutische Wirkstoffe sind erfindungsgemäß grundsätzlich alle Wirkstoffklassen wie wasserlösliche und fettlösliche, geeignet, wobei lipophile Wirkstoffe bevorzugt sind. Beispiele hierfür sind: Antihistaminika, Antiphogistika, Antibiotika, Antimykotika, Virostatika, die Durchblutung fördernde Wirkstoffe, Keratolytika, Keratoplastika, Hormone, Steroide, Vitamine u.a.

Es ist vorteilhaft, den Zubereitungen im Sinne der vorliegenden Erfindung weitere anti-irritative oder anti-entzündliche Wirkstoffe zuzugeben, insbesondere Bisabolol und/oder Panthenol, Glycyrrhetinsäure und deren Derivate, Hydrocortison-17-Valerat und deren Derivate.

Medizinische topische Zusammensetzungen im Sinne der vorliegenden Erfindung enthalten in der Regel ein oder mehrere Medikamente in wirksamer Konzentration.

### Beispiele

Die Erfindung wird anhand der nachfolgenden Beispiele näher beschrieben.

Die erfindungsgemäßen Emulsionen werden hergestellt, indem die Tenside, Alkohol, sofern vorhanden, Emulgator der Komponente c), ggf. Zusatzstoffe gemischt und bei Raumtemperatur unter-Rühren-mit der Ölphase vereinigt werden. Anschließend-werden bei gleicher Temperatur unter Rühren die Wasserphase, enthaltend ggf. wasserlösliche Wirkstoffe und / oder Zusatzstoffe, hinzugegeben und solange gerührt, bis eine klare. Lösung erhalten wurde. Die Partikel (Mizellen -) - Größe beträgt 50 bis 400, insbesondere 20 bis 300 nm.

Alle Verbindungen oder Komponenten, die in den kosmetischen Formulierungen verwendet werden können sind entweder bekannt und käuflich erwerbbar oder können nach bekannten Methoden synthetisiert werden. Alle %-Angaben sind Gewichtprozent (W/W), sofern nichts anderes angegeben.

**Beispiel 1** Primäre W/O-Typ Mikro - Emulsion.

**Tabelle 1 (Angaben in Gew. %)**

| | **1** | **2** | **3** | **4** | **5** | **6** |
|---|---|---|---|---|---|---|
| Wasser | 10,0 | 5,0 | 10 | 10,0 | 10,0 | 9,7 |
| Tween 80 (O/W) | 22,7 | 22,7 | 26,0 | 25,0 | 25,0 | 22,0 |
| Polyglycerin-2-Oleat | | | | | 10 | |
| Span 80 | 11,4 | 11,4 | 8,0 | 9,0 | | 11,0 |
| Ethanol, 96% | 4,5 | | | | | 3,0 |
| Phosphatidylcholin | 1,4 | 1,5 | | | | |
| 1,8-Octandiol | | | | | 10,0 | |
| NAT-8539 (Rhone-Poulenc) | | | 2,0 | 3,0 | | 2,0 |
| Propanol | | 5,0 | | 3,0 | | |
| Ethyloleat | | 50,4 | | | | |
| Isopropylmyristat (IPM) | 50,0 | | 50,0 | | | 52,0 |
| Myritol-318 | | | | 45,0 | 45,0 | |
| Diethylenglykolmonoethylether | | 4,0 | 4,0 | | | |
| DMSO | | | | 5,0 | | |
| Wasserstoffperoxyd, 33 %- ig | | | | | | 0,3 |

### Beispiel 2 Primäre W/O- Mikro - Emulsion mit Naturölen oder deren Gemischen

**Tabelle 2**

| | **1** | **2** | **3** | **4** | **6** |
|---|---|---|---|---|---|
| Wasser | 10,0 | 5,0 | 10,0 | 10,0 | 10,0 |
| Mandelöl/IPM (30/70) | 49,0 | | | | |
| Olivenöl/Ethyloleat (30/70) | | 52,6 | | | |
| Jojobaöl / Myritol (30/70) | | | 49,0 | | |
| Mandelöl/Avocadoöl/IPM (15/15/70) | | | | 52,0 | 41,0 |
| Tween 80 | 23,0 | 22,0 | 25,0 | 22,0 | 33,0 |
| Span 80 | 11,5 | 11,4 | 8,0 | 9,0 | 11,0 |
| Ethanol, 96% | 5,0 | 5,0 | 5,0 | 5,0 | 5,0 |
| Phosphatidylcholin | 1,5 | | | 2,0 | |
| NAT-8539 (Rhone-Polenc) | | 4,0 | 3,0 | | |

### Beispiel 3 Primäre W/O- Mikro - Emulsion mit etherischen Ölen

Die W/O-Mikro - Emulsionen wurden wie in Beispiel 1, Formulierung Nr. 1 (Tabelle 1) und Formulierung Nr. 2 (Tabelle 2) hergestellt, wobei anstatt IPM Öl-Mischungen aus etherischen Ölen (10%) in IPM, Ethyl- Oleat oder ihre Mischungen mit NaturÖlen genommen. Es wurden die folgenden etherischen Öle, separat oder als beliebige Mischungen eingesetzt:
Citrus- Öl, Pinie, Lavendelöl, Ylang Ylang, Kamille, Ravensara, Ysop, Niaouli, Anis, Nelke, Thymian, Patchuli, Weihrauch, Scharfgabe, Thuja, Melisse, Eucaliptus, Rosa Rubignose, Innophylum callophylum, als auch 1,4- Cineol, Menthol und Limonen.

### Beispiel 4 Sekundäre O/W- Mikro - Emulsion mit etherischen Ölen

Ein Teil der die etherischen Öle enthaltenden primären W/O -Mikro - Emulsion aus Beispiel 3 wurden mit 5 Teilen Wasser oder wässrigen Lösungen oder Hämoglobin - Lösungen gemischt. Dadurch wurde eine klare opake 0/W-Mikro - Emulsion mit Partikelgröße von 100 bis 200 nm erhalten.

### Beispiel 5 Primäre W/O-Mikro - Emulsion mit ungesätigen Fettsäuren

Die W/O-Mikro - Emulsionen wurden wie in Beispiel 1 Nr. 1 (Tabelle 1) und Nr. 1-(Tabelle 2) hergestellt, wobei anstelle IPM Öl-Mischungen aus ungesättigten Fettsäuren (10%) in IPM oder Ethyloleat genommen. Es wurden folgende ungesättigte Fettsäuren, separat oder als beliebige Mischungen eingesetzt, nämlich Linol- und Linolensäure, Boretschöl und Hagebutten - Öl.

### Beispiel 6. W/O- Mikro - Emulsion mit fettlöslichen Vitaminen und Provitaminen

Folgende Vitamine wurden separat oder als Mischungen in einem Öl, bzw. IPM, Myritol, Ethyloleat in Konzentrationen von 0,01 bis 10 % gelöst. Die primäre W/O-Mikro - Emulsionen wurden durch Zugabe der Tenside / Emulgator /Alkohol -Lösung und einschließend Wasser oder wässrige Lösungen , so wie im Beispiel 1 (Nr. 1 bis 5) beschrieben, gemischt. Die Partikelgröße der W/O-Mikro-Emulsion liegt im Bereich von 50 bis 100 nm.

### Beispiel 7 O/W- Mikro - Emulsion mit fettlöslichen Vitamine und Provitaminen

Ein Teil der primären Mikro - Emulsion gemäß Beispiel 5 wurde mit 5 Teilen Wasser oder wässrige Lösungen gemischt. Dadurch wurde eine klare opake O/W-Mikro - Emulsions-Lösung mit einer Partikelgröße von 100 - 200 nm erhalten.

### Beispiel 8 Hämoglobin-haltige W/O-Mikro-Emulsion

Hämoglobin Vorbereitung: Antioxidantien: L-Cystein und N-AC-Cystein, (0,1-0,03%), Glukose (1,0 %), Konservierungsmittel: Salicylat Na (K) (0.25%)..

Die Hb- Lösung wurde mit CO-Gas 30 Minuten begast, so dass der Hb-CO-Gehalt mehr als 98% des gesamten Hb beträgt.

Die Zusammensetzungen wie in Tabelle 3 angegeben wurden durch Mischung der Bestandteile der primären W/O-Mikro - Emulsion (Basis-ME) gemäß Beispiel 1,Tabelle 1, Nr.1, Nr.2 und 6) und der o.g. Hämoglobin- Lösung bei Raumtemperatur unter vorsichtigem Rühren bis zur Entstehung einer W/O-Mikro - Emulsion als ein klares Gel oder eine O/W-Mikro - Emulsion als Lösung mit einer Partikelgröße von 200 bis 300 nm hergestellt.

**Tabelle 3 (Mengenangaben in ml)**

| | 1 | 2 | 3 | 4 | 5 |
|---|---|---|---|---|---|
| Basis ME Nr.1 | 6,0 | | | 6,0 | 1,0 |
| Basis ME Nr.2 | | 6,0 | | | |
| Basis ME Nr.6 | | | 6,0 | | |
| Hämoglobin Lösung, 30% | 3,0 | | | | |
| Hämoglobin Lösung, 25 % | | 3,0 | | | 5,0 |
| Hämoglobin Lösung, 10 % | | | 3,0 | | |
| Hömoglobin-Lösung 5% | | | | 3,0 | |
| Produktform | Transparentes Gel | Transparentes Gel | Transparentes Gel | Transparentes Gel | Lösung |

### Beispiel 9 W/O ME zur Hautbehandlung mit NMF(wasserbindende Stoffe), Vitaminen und kosmetischen Zusatzstoffen

Die Zusammensetzungen wurden durch Kombination von primären W/0- Mikro-Emulsion, wie beschrieben, und einer NMF- Lösung unter vorsichtigem Rühren bei Raumtemperatur bis zu Entstehung einer klaren W/O-Mikro-Emulsion mit Partikelgröße von 50 bis 300 nm hergestellt. Als Wirkstoffe wurden eingesetzt: Sorbitol (0,5-5,0 %), Allantoin (0,2-0,5), Glycerin (0,5-10,0), PCA-Na (0,5-5,0), Harnstoff (0,5-20,0), Aminosäuren oder Proteinhydrolysate (Seiden- Protein, Weizenkeim-Protein, Hefe) (0,1-5,0), Oligo- Saccharide (Trehalose, Chito-Oligosaccharide,) (0,1-5,0), wasserlösliche Vitamine und Modifikate (Vitamin C, Vitamin H, (0,01-0,5), Hämoglobin - Lösung sowie als Zusatz anorganische Salze.

**Tabelle 4 (Angaben in Gramm)**

| | 1 | 2 | 3 | 4 |
|---|---|---|---|---|
| Basis ME Nr. 2 (Tabelle 1) | 60,0 | | 60,0 | |
| Basis ME Nr. 4 (Tabelle 1) | | 60,0 | | 60,0 |
| Wasser | 20,8 | 25,75 | 24,6 | 24,8 |
| Sorbitol | 1,0 | 1,0 | 1,0 | 1,0 |
| Allantoin | 0,15 | 0,15 | 0,1 | |
| Pyrrolidoncarbonsäure Na-Saltz | 1,0 | 1,0 | 0,5 | 1,0 |
| Chitooligosaccharide | 0,3 | | 1,0 | 1,0 |
| Harnstoff | 1,5 | 1,0 | 1,5 | 1,0 |
| Glycerin | | 0,8 | | |
| Aloe Vera, 100% | 0,1 | | 0,3 | 0,3 |
| Ascorbinsäure Na | | | 0,01 | |
| Biotin | | | | 0,5 |
| Seidenprotein | 0,2 | | 0,5 | 0,3 |
| Hydrolysat | | | | |
| Hydrolysierte Kollagen (Crotein D) | | 0,3 | | 0,3 |
| Hyaluronsäure | | 0,01 | | |
| NaCl | 0,1 | | 0,1 | |
| Hämoglobin | | 0,02 | 0,02 | 0,02 |

### Beispiel 10 O/W- Mikro-Emulsion für Hautbehandlung mit NMF(wasserbindende Stoffe), Vitaminen und kosmetische Zusatzstoffen

Ein Teil der primären W/O- Mikro - Emulsion aus Beispiel 9 (Nr. 1) wurde mit 5 Teilen Wasser oder einer wässrigen Lösung gemischt. Dadurch wurde eine klare opake ME-Lösung mit einer Partikelngröße von 100 bis 200 nm erhalten.

### Beispiel 11 Pflanzliche und (oder) mikrobiologische Extrakte enthaltende W/O-Mikro-Emulsion

Folgende wässerigen oder alkoholischen Extrakte oder deren Mischungen, nämlich: Aloe Vera, Sonnenhut (Echinacea), Grüner Tee, Hammamelis Extrakt, Meristeme Extrakt, Niembaum, Polyplant ME (Polygon Chemie,CH) wurden separat oder als Mischungen als wässrige Phase genommen und mit einer Öl-Phase gemischt (wie Nr. 1 bis Nr. 5, Tabelle 4).

### Beispiel 12. Herstellung einer pflanzliche und (oder) mikrobiologische Extrakte enthaltenden O/W-Mikro-Emulsion

Ein Teil der primären W/O- Mikro - Emulsion Tabelle 1 (Nr. 1 aus Beispiel 1) wurde mit 5 Teilen pflanzlichen Extrakten und Hb- Lösung gemischt. Dadurch wurde eine klare opake Mikro - Emulsions - Lösung mit einer Partikelgröße von 100 bis 200 nm erhalten.

### Beispiel 13 W/O-Mikro - Emulsion mit Polysacchariden und Oligosacchariden

Oligosaccharide (Trehalose, Chito- Oligosaccharide), Polysaccharide, Hyaluronsäure, Chitosan, Xanthan, Aloe Vera,) wurden als wässrige Phase genommen und wie in Beispiel 11 zu einer ME formuliert.

### Beispiel 14. O/W-Mikro - Emulsion mit Polysacchariden und Oligosacchariden

Ein Teil der primären W/O- Mikro - Emulsion aus Beispiel 13 wurde mit 5 Teilen Wasser oder wässriger Lösung und Hb- Lösung gemischt. Dadurch wurde eine klare opake Mikro- Emulsions- Lösung mit einer Partikelngröße von 100 bis 200 nm erhalten.

### Beispiel 15. W/O -Mikro - Emulsion zur Selbstbräunung

Die Zusammensetzung wurden durch Kombination von zwei Teilen der primären Mikro-Emulsion (Beispiel 1, Tabelle 1, Nr 1) und einem Teil einer 5%-igen wässrigen Lösung von 1,3- Dihydroxypropan-2-on hergestellt.

### Beispiel 16 W/O Mikro - Emulsion zur Depigmentierung

Hier wurden folgende Stoffe inkorporiert in eine Basis Mikro- Emulsion gemäß Beispiel 1, Tabelle 1, Nr 1 bis 5:Hydrochinon und Derivate, Azelaïnsäure,4-Isopropylkatechol, Naturstoffen wie Ascorbinsäure reiche Pflanzen (Petersilie, Zitronensaft, Hagebutten, Schwertlilien).

### Beispiel 17 W/O -Mikro - Emulsion mit Superoxid - Dismutase und Katalase

In eine Basis Mikro-Emulsion gemäß Beispiel Beispiel 8, Tabelle 3 Nr. 1 bis 5 anstatt Hämoglobin-Lösung wurde Superoxid- Dismutase (SOD), rekombinante Human Superoxid- Dismutase aus Hefe (Resbio Ltd, Russland), Katalase oder Hämoglobin/Superoxid -. Dismutase und Hämoglobin/ Katalase inkorporiert. Die Menge von Katalase und Superoxid- Dismutase beträgt von 0,01 bis 0,3 %.

### Beispiel 18. W/O-Mikro - Emulsion mit Antioxidantien.

In eine Mirkoemulsion gemäß Beispiel Tabelle 3, Nr 1 bis 5 wurde in die wässrige

Phase Melatonin Ascorbinsäure Cystein und N-Acetyl-Cystein eingearbeitet.

### Beispiel 19 Dermatologisch-medizinische W/O und O/W Mikro - Emulsionen

Zwei Teile der primären Mikro - Emulsion gemäß Beispiel 1 , Nr.1 bis 5 wurden mit einem Teil Wirkstofferhaltende wässrige Lösungen (Tabelle 5)) gemischt. Dadurch wurde eine klare opake W/O- Mikro - Emulsion mit einer Partikelngröße von 100 - 300 nm erhalten.

**Tabelle 5**

| Basis ME | Wirkstoff 1 | Wirkstoff 2 | Anwendung |
|---|---|---|---|
| Beispiel 1 Nr.1 bis 5 | Glycerrhytinsäure Zn-Salz | Melatonin | Akne |
| Beispiel 1, Nr. 1 bis 5 | Glycerrhytinsäure, | Borretschöl | Neurodermitis |
| Beispiel 1 Nr. 1 bis 5 | Harnstoff | Totes- Meer- Salz | Neurodermitis |
| Beispiel 1, Nr. 1 bis 5 | Dihydroxyaceton | | Vitiligo |

### Beispiel 20 Nachweis der Eindringtiefe von Mikro - Emulsionen in die Haut

Die Eindringtiefe erfindungsgemäßer Mikro - Emulsionen wurde im Rahmen eines Schälverfahrens an einer Modell-Haut bestimmt. Schweine-Ohren wurden ohne Hautverietzungen sofort nach der Schlachtung aus einem Schlachthof abgeholt. Die Ohren wurden gründlich mit milder Seife abgewaschen. Danach folgte eine milde Schälung mit 2 %-iger Glukolsäure im Laufe von 15 Minuten. Anschließend wurde die Haut mit 2%-ger Natrium- Hydrokarbonat- Lösung neutralisiert und mit Wasser abgespült. Währenddessen wurden die Ohren bei 35°C temperiert.

Folgende Mikro - Emulsionen wurden auf die Hautoberfläche der Ohren aufgetragen und etwa 1 bis 2 Minuten in die Haut einmassiert
1. Hämoglobin- haltige ME gemäß Beispiel 8, Nr. 3
2. NMF- haltige W/0- ME gemäß Beispiel 9 Nr.1
3. NMF- haltige O/W- ME gemäß Beispiel 10
4. Kontrolle ME gemäß Beispiel , Nr. 1.

Danach ließ man sie bei 35 °C etwa 30 Minuten einwirken. Anschließend wurden die Reste der Mikro - Emulsion mit milder Seife abgewaschen und die Haut abgetrocknet.

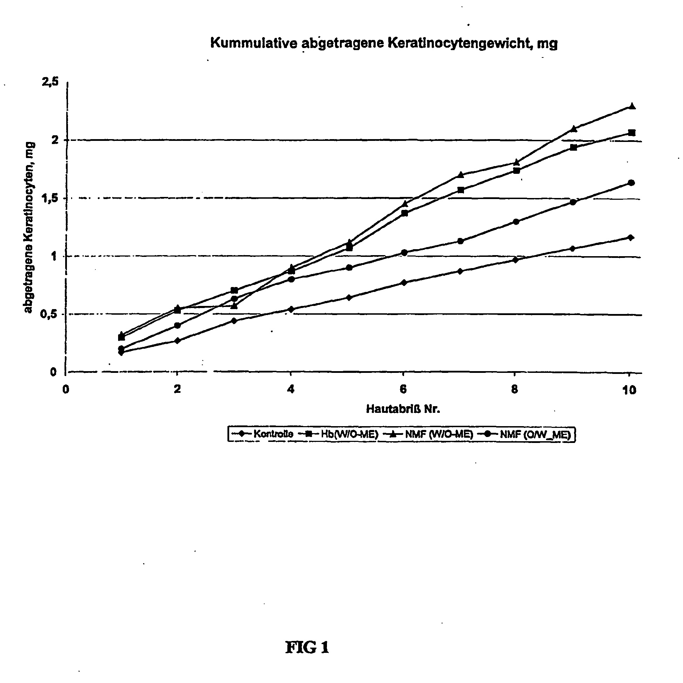

Für die Schälung wurden 15 Tesafilmstreifen (Tesafilm, Beiersdorf) auf eine Größe von 1,5 cm * 1,5 cm geschnitten und ausgewogen. Die auf dem Schweine-Ohr ausgewählten Untersuchungsfelder von 2 cm * 2 cm wurden nacheinander mit den vorbereiteten Tesafilm-Streifen beklebt und danach abgezogen. Die Streifen wurden erneut gewogen. Die abgetragene Menge von Keratinocyten des Stratum corneums ermittelte man als Differenz zwischen den Streifen-Gewichten vor und nach der Abtragung. Das kumulative Gewicht von abgetragenen Keratinocyten wurde gegen die Abtragungsschritte in ein Diagramm aufgetragen (Fig.1).

Wie hieraus ersichtlich ist, erfolgt mit der erfindungsgemäßen Mikro-Emulsion als solcher eine erhebliches Eindringen in die tieferen Hautschichten, so dass Wirkstoffe besser und effektiver agieren können.

Im folgenden Beispiel 21 ist der Nachweis der Wirksamkeit von erfindungsgemäß eingesetzten Wirkstoffen aufgezeigt

### Beispiel 21. Nachweis der erleichterten Sauerstoff-Diffusion in die Haut mittels Hämoglobinhaltiger W/O-Mikro - Emulsion.

Der Nachweis der erleichterten Sauerstoff-Diffusion in die Haut wurde am menschlichen Unterarm durchgeführt. Zuerst wurde der Unterarm mit milder Seife gereinigt. Darauf folgt eine leichte Schälung entweder mit 2%-iger Glykolsäure oder mehrmals mit Tesafilmstreifen (s.o.).

Die Hämoglobinhaltige W/O Mikro - Emulsionen (Nr. 3 und Nr.5, Beispiel 8) wurden auf die vorbereitete Hautoberfläche eingetragen und leicht einmassiert und für 15 Minuten einwirken gelassen. Als Kontrolle wurde leicht geschälte Haut gemessen. Danach wurde die Haut abgewaschen und abgetrocknet. An der Mess - Stelle wurde eine Silikon-Membran aufgeklebt, die als Messschicht RuCl Hexahydrat- enthält. Die Fluoreszenzabnahme an der Membran Als maß für den Sauerstoffpartialdruck pO₂ wurde nach Verfahren (M. Stücker, P. Altmeyer et al., The Transepidermal Oxygen Flux from the Environment is in Ballance with the Capillary Oxygen Supply //J. Investigative Dermatology 2000, Vol. 114, NO 3, p.533-540) im Laufe von 90 Minuten kontinuierlich gemessen. Die Sauerstoff- Aufnahme der lebenden Zellen in der Epidermis als Anfangsabfall der pO2- Werte wurde ermittelt.

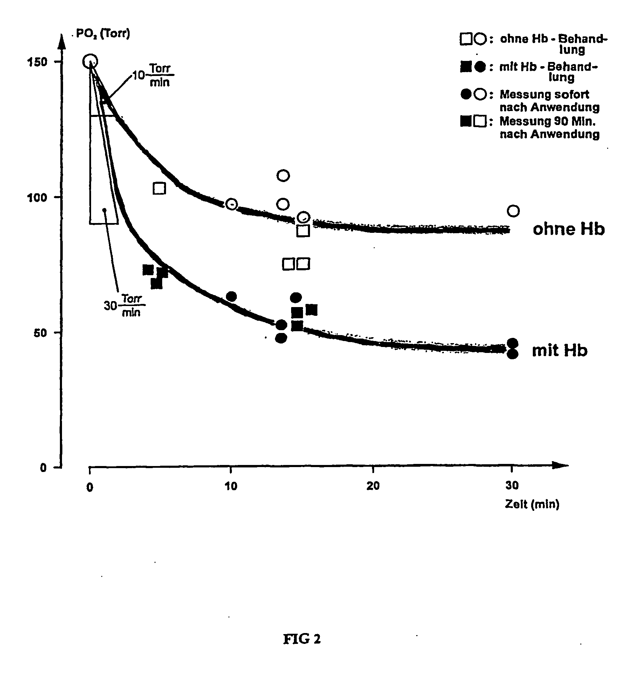

In Fig. 2 ist das Ergebnis dieser Messungen dargestellt. Wie hieraus ersichtlich, ist, zeigt die Hämoglobinhaltige Mikro - Emulsion eine 3 - fache Erhöhung der Sauerstoff - Aufnahme gegenüber der Kontrolle.

### Beispiel 22. Nachweis der Haut-Verträglichkeit der Hämoglobinhaltigen W/O-Mikro - Emulsionen.

Für den Epikutantest, der als Nachweis für eine primäre Hautirritation oder für eine Kontaktallergie dient, wurde Hämoglobin - haltige W/O-Mikro - Emulsion (Beispiel 8, Tabelle 3, Nr.3) in einer Menge von 2 bis 5 mg/cm² Haut verwendet. Ein Kollektiv von 32 weiblichen und 18 männlichen Probanden im Alter von 18 bis 69 Jahren nahm an dem Test teil, darunter 10 Allergiker und 13 hautempfindliche Probanden.

Die o.g. Mikro - Emulsion wird unter Verwendung eines handelsüblichen Testpflasters auf die klinisch gesunde Haut aufgelegt und fixiert. Das Testpflaster wird nach 48 Stunden entfernt und das Testfeld beurteilt. Eine weitere Beurteilung erfolgt nach 72 Stunden.

Es konnten bei keinem Probanden weder nach 48 noch nach 72 Stunden positive oder zweifelhafte Reaktionen nachgewiesen werden, so dass sich bei dieser Prüfung kein Hinweis dafür ergab, dass die Mikro - Emulsion in der vorliegenden Testkonzentration auf die Haut primär irritierend wirkte. Auch ließ sich bei diesem Test keine bereits bestehende Sensibilisierung durch Inhaltstoffe der Mikro - Emulsion auslösen.

### Beispiel 23. Nachweis der Wirksamkeit der Kosmetikbehandlung mit der Hämoglobinhaltigen W/O-Mikro - Emulsion.

Die kosmetische Behandlung mit der Hämoglobinhaltigen W/O-Mikro - Emulsion (Beispiel 8, Tabelle 3, Nr.3) fand über 8 Wochen an insgesamt 14 freiwilligen Probandinnen mit trockener, leicht gealterter Haut statt. Vor Beginn der Behandlung sowie nach der 8-wöchigen Behandlung wurden alle Probandinnen mittels des sogenannten SELS-Verfahrens (Surface Evaluation of Living Skin) vermessen. Es wurden folgende Hautparameter gemessen: Rauhigkeit, Schuppigkeit, Glätte und Faltigkeit. Die Messungen fanden an der Stirn, im Augenwinkelbereich sowie im Mundwinkelbereich statt.

Es wurde festgestellt, dass generell eine sichere hautglättende Wirkung bei 13 ausgewerteten Probandinnen nachgewiesen werden konnte. Dies betrifft im wesentlichen die Faltigkeit: Abnahme um 29 % (Stirn), 43 % (Mundwinkel) und 17% (Augenwinkel) im Vergleich zum Anfang der Behandlung. Ebenso die Hautglätte wurde verbessert um 39 % (Stirn), 72 % (Mundwinkel) und 74 % (Augenwinkel) im Vergleich zum Anfang der Behandlung.

## Patentansprüche

1. Wasser - in Öl - Mikro - Emulsion zur topischen Anwendung mit binärer Phasen- und Wirkstoffdifferenzierbarkeit, **dadurch gekennzeichnet, dass** sie Vernetzerfrei ist und
a) 45 bis 90 Gew. % einer flüssigen Ölphase,
b) 5 bis 40 Gew. % einer Mischung aus einem oder mehreren nicht ionischen W/O- Tensiden mit einem HLB- Wert von 3 bis 7 und einem oder mehreren nicht ionischen O/W- Tensiden mit einem HLB- Wert von 9 bis 18 im Verhältnis 1:4 bis 1:1,2 ;
c) 0,01 bis 20 Gew. % eines oder mehrerer Phospholipide; Cholesterine, Cholesterin-Derivate;
d) 0,00 bis 15 Gew. % eines oder mehrerer ein- oder zweiwertigen C₁₋₈-Alkohole;
e) 1 bis 10 Gew. % Wasser oder wässrige Lösungen
aufweist, wobei die Mizellen dieser primären Mikro- Emulsion eine Partikelgröße von 20 bis 400 nm aufweisen und die Emulsion durch Umsetzung mit einer Wasserphase wahlweise in eine sekundäre W/O- Mikro-Emulsion oder in eine O/W- Mikro - Emulsion konvertierbar ist.

2. Wasser -in Öl- Mikro-Emulsion gemäß Anspruch 1, **dadurch gekennzeichnet, dass** sie 0 bis 30 Gew. % eines oder mehrerer wasserlöslicher oder fettlöslicher Wirkstoffe oder Mischungen von Wasser- und Fettlöslichen Wirkstoffen enthält.

3. Wasser- in Öl- Mikro-Emulsion gemäß Anspruch 2, **dadurch gekennzeichnet, dass** sie 0,001 bis 20 Gew. % eines oder mehrerer biologischer Sauerstoffträger aufweist.

4. Wasser -in Öl- Mikro-Emulsion gemäß einem der Ansprüche 1 bis 3, **dadurch gekennzeichnet, dass** zusätzlich 0 bis 15 Gew. % Zusatzstoffe vorhanden sind.

5. Wasser -in Öl- Mikro-Emulsion gemäß Anspruch 1 bis 4 **dadurch gekennzeichnet, dass** sie
a) 50 bis 80 Gew. % einer Ölphase
b) 5 bis 40 Gew. % einer Mischung aus einem oder mehreren W/O- und einem oder mehreren O/W- Tensiden;
c) 0,1 bis 10 Gew. % eines oder mehrerer Lecithine, Phosphatidylcholine oder Derivaten oder Mischungen hiervon;
d) 0,0 bis 10 Gew. % eines oder mehrerer ein- oder zweiwertigen C₁₋₈- Alkohols und
e) 1 bis 10 Gew. % Wasser oder wässrigen Lösungen aufweist.

6. Wasser- in - Öl- Mikro - Emulsion gemäß einem der Ansprüche 1 bis 5, **dadurch gekennzeichnet, dass** sie 0,01 bis 15 Gew. % eines oder mehrerer Alkohole gemäß d) enthält.

7. Wasser- in- Öl- Mikro-Emulsion gemäß einem der Ansprüche 1 bis 6, **dadurch gekennzeichnet, dass** das Lecithin, Phosphatidylcholin oder Derivat hiervon ausgewählt ist aus Phosphatidylcholin, Sojalecithin, Eilecithin, Gemischen aus Phosphatidylcholin und Lecithin in unterschiedlichen Anteilen oder Mischungen hiervon.

8. Wasser- in- Öl- Mikro-Emulsion gemäß einem der Ansprüche 1 bis 7 , **dadurch gekennzeichnet, dass** der Alkohol ausgewählt ist aus Ethanol, Isopropanol, Butanol, 1,6-Oktandiol, 1,2-Hexandiol.

9. Wasser- in- Öl- Mikro-Emulsion gemäß einem der Ansprüche 1 bis 8, **dadurch gekennzeichnet, dass** sie wasserlösliche Wirkstoffe enthält, die ausgewählt sind aus Aminosäuren, Peptiden, Protein-Hydrolysaten, Proteinen, Sacchariden, Oligosacchariden, Polysacchariden und Derivaten, Hormonen und Hormonähnlichen Stoffen, Antioxidantien, Vitaminen und Provitaminen, AHA-Säuren, NMF, Oxidationsmitteln, pflanzlichen Extrakten, Flavonoiden und pflanzlichen Polyphenolen oder Mischungen hiervon.

10. Wasser- in- Öl- Mikro-Emulsion gemäß einem der Ansprüche 1 bis 9, **dadurch gekennzeichnet, dass** sie fettlösliche Wirkstoffe enthält, die ausgewählt sind aus Antioxidantien, Vitaminen und Provitaminen, ungesättigten Fettsäuren, Ceramiden , ätherische Ölen oder Mischungen hiervon.

11. Wasser- in- Öl- Mikro-Emulsion gemäß einem der Ansprüche 1 bis 10, **dadurch gekennzeichnet, dass** sie dermatologisch wirksame Mittel, ausgewählt aus Hormonen und Hormon- ähnlichen Stoffen, Antymikotika, Narbenmittel, Gerbstoffe, Teere, Keratinolytika, Keratinoplastika, Photocumarinen, Azelainsäure oder Mischungen hiervon aufweist.

12. Wasser - in -Öl- Mikro- Emulsion gemäß einem der Ansprüche 1 bis 11, **dadurch gekennzeichnet, dass** als Zusatzstoffe Elektrolyte, Oxidantien, chelatierende Stoffe, Diffusionsverstärker, Penetrationsförderer, Feuchthalter oder Mischungen hiervon enthalten sind.

13. Wasser- in- Öl- Mikro-Emulsion gemäß einem der Ansprüche 1 bis 12, **dadurch gekennzeichnet, dass** sie biologische Sauerstoffträger, ausgewählt aus nativem, modifiziertem oder unmodifiziertem Hämoglobin, Myoglobin oder Mischungen hiervon in einer Gesamtmenge von 0,001 bis 20 Gew. % enthält.

14. Wasser- in- Öl- Mikro-Emulsion gemäß Anspruch 13, **dadurch gekennzeichnet, dass** sie weiterhin Antioxidantien, Glutathion, Superoxid- Dismutase, Melatonin , Flavonoide, Aminosäuren oder Mischungen hiervon enthält.

15. Wasser- in -Öl Mikro - Emulsion gemäß einem der Ansprüche 13 oder 14, **dadurch gekennzeichnet, dass** sie weiterhin Glukose enthält.

16. Wasser- in- Öl- Mikro-Emulsion gemäß einem der Ansprüche 1 bis 15, **dadurch gekennzeichnet, dass** sie zusätzlich Vitamine und Provitamine in einer Menge von 0,01 bis 1,0 Gew. % enthält.

17. Wasser- in- Öl- Mikro-Emulsion gemäß einem der Ansprüche 1 bis 16, **dadurch gekennzeichnet, dass** sie Pflanzenextrakte in einer Menge von 0,1 Gew. % bis 5 Gew. %, 0,1 Gew. % bis 5 Gew. % etherische Öle, 0,1 Gew. % bis 10 Gew. % AHA-Säuren, 0,01 bis 0,3 Gew .% Hormone oder Hormon- ähnliche Stoffe, 0,1 bis 5 Gew. % essentielle Fettsäuren, Ceramide oder Mischungen hiervon aufweist.

18. Topisch applizierbare Zubereitung in Form einer Mikro - Emulsion, **dadurch gekennzeichnet, dass** sie eine Mischung einer Zusammensetzung gemäß einem der Ansprüche 1 bis 17 oder eine Mischung hiervon mit 0,1 bis 90 Gew. % einer Wasserphase, wobei die Zubereitung dann 1 bis 50 Gew. % der Ölphase aufweist und eine W/O-oder eine O/W- Mikro - Emulsion darstellt.

19. Verfahren zur Herstellung einer Mikro- Emulsion gemäß einem der Ansprüche 1 bis 18, **dadurch gekennzeichnet, dass** man die Ölphase, ggf. mit darin vorhandenen fettlöslichen Wirkstoffen, enthaltend das oder die Tenside, das oder die Phospholipide; Cholesterine, Cholesterin-Derivate; sowie den oder die Alkohole und ggf. Zusatzstoffe, mit einer Wasserphase, und ggf. dem oder den wasserlöslichen Wirkstoffen, ggf. Zusatzstoffen, bei Temperaturen von 10 bis 30 °C miteinander vermischt, und die so erhaltene primäre W/O- Mikro- Emulsion ggf. mit einer Wasserphase, die ggf. weitere wasserlösliche Wirkstoffe, ggf. Zusatzstoffe aufweisen kann, in eine sekundäre W/O- oder eine sekundäre 0/W-Mikro- Emulsion überführt.

20. Topische, nicht-therapeutische Verwendung einer Mikro-Emulsion gemäß einem der Ansprüche 1 bis 18 als kosmetische Zubereitung für Haut oder die Pflege, Reinigung, Konditionierung von Haar.

21. Verwendung gemäß Anspruch 20, **dadurch gekennzeichnet, dass** es sich um altersbedingt veränderte Haut handelt.

22. Verwendung gemäß Anspruch 20 oder 21, **dadurch gekennzeichnet, dass** eine Zubereitung gemäß Anspruch 2, 13 bis 15 eingesetzt wird.

23. Verwendung gemäß einem der Ansprüche 20 bis 22, **dadurch gekennzeichnet, dass** die Emulsion flüssig ist und aufgesprüht wird oder als Gel aufgetragen wird.

24. Verwendung einer Mikro-Emulsion gemäß einem der Ansprüche 1 bis 18 zur Herstellung einer dermatologischen, pharmazeutischen Zubereitung zur topischen Behandlung einer allergisch, bakteriell, immunologisch, durch äußere Einflüsse beeinträchtigten, irritierten, beschädigten oder degenerierten Haut.

25. Verwendung gemäß Anspruch 24, **dadurch gekennzeichnet, dass** eine Zubereitung gemäß Anspruch 2, 13 bis 15 eingesetzt wird.

26. Verwendung gemäß einem der Ansprüche 24 oder 25, **dadurch gekennzeichnet, dass** die Emulsion flüssig ist und aufgesprüht wird oder als Gel aufgetragen wird.

## Claims

1. Water-in-oil microemulsion for topical application, with binary phase differentiability and active substance differentiability, **characterised in that** it is free from crosslinking agents and comprises
a) 45 to 90 wt.% of a liquid oily phase,
b) 5 to 40 wt.% of a mixture of one or more non-ionic W/O surfactants with an HLB value of 3 to 7 and one or more non-ionic O/W surfactants with an HLB value of 9 to 18 in a ratio of 1:4 to 1:1.2,
c) 0.01 to 20 wt.% of one or more phospholipids, cholesterols, cholesterol derivatives,
d) 0.00 to 15 wt.% of one or more monohydric or dihydric C₁₋₈- alcohols,
e) 1 to 10 wt.% of water or aqueous solutions, wherein the micelles of this primary microemulsion have a particle size of 20 to 400 nm and the emulsion can be converted if desired by reaction with an aqueous phase into a secondary W/O microemulsion or into an O/W microemulsion.

2. Water-in-oil microemulsion according to claim 1, **characterised in that** it contains 0 to 30 wt.% of one or more water-soluble or fat-soluble active substances or mixtures of water and fat-soluble active substances.

3. Water-in-oil microemulsion according to claim 2, **characterised in that** it comprises 0.001 to 20 wt.% of one or more biological oxygen carriers.

4. Water-in-oil microemulsion according to one of claims 1 to 3, **characterised in that** it additionally contains 0 to 15 wt.% of additives.

5. Water-in-oil microemulsion according to claims 1 to 4, **characterised in that** it comprises
a) 50 to 80 wt.% of an oily phase,
b) 5 to 40 wt.% of a mixture of one or more W/O and one or more O/W surfactants,
c) 0.1 to 10 wt.% of one or more lecithins, phosphatidylcholines or derivatives or mixtures thereof,
d) 0.0 to 10 wt.% of one or more monohydric or dihydric C₁₋₈- alcohols and
e) 1 to 10 wt.% of water or aqueous solutions.

6. Water-in-oil microemulsion according to one of claims 1 to 5, **characterised in that** it contains 0.01 to 15 wt.% of one or more alcohols according to d).

7. Water-in-oil microemulsion according to one of claims 1 to 6, **characterised in that** the lecithin, phosphatidylcholine or derivative thereof is selected from phosphatidylcholine, soya lecithin, eilecithin, mixtures of phosphatidylcholine and lecithin in different proportions or mixtures thereof.

8. Water-in-oil microemulsion according to one of claims 1 to 7, **characterised in that** the alcohol is selected from ethanol, isopropanol, butanol, 1,6-octanediol, 1,2-hexanediol.

9. Water-in-oil microemulsion according to one of claims 1 to 8, **characterised in that** it contains water-soluble active substances that are selected from amino acids, peptides, protein hydrolysates, proteins, saccharides, oligosaccharides, polysaccharides and derivatives, hormones and hormone-like substances, anti-oxidants, vitamins and provitamins, AHA acids, NMF, oxidising agents, plant extracts, flavonoids and plant polyphenols or mixtures thereof.

10. Water-in-oil microemulsion according to one of claims 1 to 9, **characterised in that** it contains fat-soluble active substances that are selected from anti-oxidants, vitamins and provitamins, unsaturated fatty acids, ceramides, ethereal oils or mixtures thereof.

11. Water-in-oil microemulsion according to one of claims 1 to 10, **characterised in that** it comprises dermatologically active agents selected from hormones and hormone-like substances, antimycotics, scar healing agents, tannins, tars, keratinolytics, keratinoplastics, photocoumarins, azelaic acid or mixtures thereof.

12. Water-in-oil microemulsion according to one of claims 1 to 11, **characterised in that** it contains additives selected from electrolytes, oxidising agents, chelating agents, diffusion enhancers, penetration promoters, moisturisers or mixtures thereof.

13. Water-in-oil microemulsion according to one of claims 1 to 12, **characterised in that** it contains biological oxygen carriers selected from native, modified or unmodified haemoglobin, myoglobin or mixtures thereof in a total amount of 0.001 to 20 wt.%

14. Water-in-oil microemulsion according to claim 13, **characterised in that** it also contains anti-oxidants, glutathione, super oxide dismutase, melatonin, flavonoids, amino acids or mixtures thereof.

15. Water-in-oil microemulsion according to one of claims 13 and 14, **characterised in that** it furthermore contains glucose.

16. Water-in-oil microemulsion according to one of claims 1 to 15, **characterised in that** it additionally contains vitamins and provitamins in an amount of 0.01 to 1.0 wt.%.

17. Water-in-oil microemulsion according to one of claims 1 to 16, **characterised in that** it comprises plant extracts in an amount of 0.1% wt.% to 5 wt.%, 0.1 wt.% to 5 wt.% of ethereal oils, 0.1 wt.% to 10 wt.% of AHA acids, 0.01 wt.% to 0.3 wt.% of hormones or hormone-like substances, 0.1 wt.% to 5 wt.% of essential fatty acids, ceramides or mixtures thereof.

18. Topically applicable preparation in the form of a microemulsion, **characterised in that** it is a mixture of a composition according to one of claims 1 to 17 or a mixture thereof with 0.1 to 90 wt.% of an aqueous phase, in which the preparation then contains 1 to 50 wt.% of the oily phase and forms a W/O or a O/W microemulsion.

19. Process for the preparation of a microemulsion according to one of claims 1 to 18, **characterised in that** the oily phase, optionally with fat-soluble active substances present therein and containing the surfactant or surfactants, the phospholipid or phospholipids, cholesterols, cholesterol derivatives, as well as the alcohol or alcohols and optionally additives, is mixed with an aqueous phase and optionally with the water-soluble active substance or substances, and optionally additives, at temperatures from 10° to 30°C, and the primary W/O microemulsion thereby obtained is optionally converted with an aqueous phase, which may optionally comprise further water-soluble active substances and optionally additives, into a secondary W/O or a secondary O/W microemulsion.

20. Topical, non-therapeutic use of a microemulsion according to one of claims 1 to 18 as a cosmetic preparation for the skin or for the care, cleaning and conditioning of hair.

21. Use according to claim 20, **characterised in that** the use is for skin affected by ageing.

22. Use according to claim 20 or 21, **characterised in that** a preparation according to claim 2, 13 to 15 is used.

23. Use according to one of claims 20 to 22, **characterised in that** the emulsion is liquid and is sprayed on or applied as a gel.

24. Use of a microemulsion according to one of claims 1 to 18 for the preparation of a dermatological, pharmaceutical preparation for the topical treatment of skin that has been injured, irritated, damaged or has degenerated due to allergic, bacterial, immunological or external influences.

25. Use according to claim 24, **characterised in that** a preparation according to claim 2, 13 to 15, is used.

26. Use according to one of claims 24 and 25, **characterised in that** the emulsion is liquid and is sprayed on or applied as a gel.

## Revendications

1. Microémulsion de type eau dans l'huile destinée à l'application topique avec une différentiabilité de phase binaire et de substance active, **caractérisée en ce qu'**elle ne contient pas de réticulant et contient
a) 45 à 90 % en masse d'une phase huileuse liquide,
b) 5 à 40 % en masse d'un mélange constitué d'un ou plusieurs agents tensioactifs de type eau dans l'huile non ioniques ayant une valeur HLB de 3 à 7 et d'un ou plusieurs agents tensioactifs de type huile dans l'eau non ioniques ayant une valeur HLB de 9 à 18, dans un rapport de 1:4 à 1:1,2;
c) 0,01 à 20 % en masse d'un ou plusieurs phospholipides, cholestérols, dérivés de cholestérol;
d) 0,00 à 15 % en masse d'un ou plusieurs mono- ou dialcools en C₁-C₈;
e) 1 à 10 % en masse d'eau ou de solutions aqueuses,
les micelles de cette microémulsion primaire ayant une taille de particules de 20 à 400 nm et l'émulsion étant transformable au choix en une microémulsion de type eau dans l'huile secondaire ou en une microémulsion de type huile dans l'eau par réaction avec une phase aqueuse.

2. Microémulsion de type eau dans l'huile selon la revendication 1, **caractérisée en ce qu'**elle contient 0 à 30 % en masse d'une ou plusieurs substances actives solubles dans l'eau ou solubles dans les graisses ou de mélanges de substances actives solubles dans l'eau et dans les graisses.

3. Microémulsion de type eau dans l'huile selon la revendication 2, **caractérisée en ce qu'**elle contient 0,001 à 20 % en masse d'un ou plusieurs transporteurs d'oxygène biologiques.

4. Microémulsion de type eau dans l'huile selon l'une des revendications 1 à 3, **caractérisée par** la présence supplémentaire de 0 à 15 % en masse d'additifs.

5. Microémulsion de type eau dans l'huile selon les revendications 1 à 4, **caractérisée en ce qu'**elle contient
a) 50 à 80 % en masse d'une phase huileuse,
b) 5 à 40 % en masse d'un mélange constitué d'un ou plusieurs agents tensioactifs de type eau dans l'huile et d'un ou plusieurs agents tensioactifs de type huile dans l'eau;
c) 0,1 à 10 % en masse d'une ou plusieurs lécithines, phosphatidylcholines ou de leurs dérivés ou de leurs mélanges;
d) 0,0 à 10 % en masse d'un ou plusieurs mono- ou dialcools en C₁-C₈; et
e) 1 à 10 % en masse d'eau ou de solutions aqueuses.

6. Microémulsion de type eau dans l'huile selon l'une des revendications 1 à 5, **caractérisée en ce qu'**elle contient 0,01 à 15 % en masse d'un ou plusieurs alcools selon d).

7. Microémulsion de type eau dans l'huile selon l'une des revendications 1 à 6, **caractérisée en ce que** la lécithine, la phosphatidylcholine ou leur dérivé est choisi parmi la phosphatidylcholine, la lécithine de soja, la lécithine d'oeuf, des mélanges de phosphatidylcholine et de lécithine en différentes proportions ou leurs mélanges.

8. Microémulsion de type eau dans l'huile selon l'une des revendications 1 à 7, **caractérisée en ce que** l'alcool est choisi parmi l'éthanol, l'isopropanol, le butanol, le 1,6-octanediol, le 1,2-hexanediol.

9. Microémulsion de type eau dans l'huile selon l'une des revendications 1 à 8, **caractérisée en ce qu'**elle contient des substances actives solubles dans l'eau qui sont choisies parmi des aminoacides, des peptides, des hydrolysats de protéines, des protéines, des saccharides, des oligosaccharides, des polysaccharides et leurs dérivés, des hormones et des substances analogues à des hormones, des antioxydants, des vitamines et des provitamines, des acides AHA, des NMF, des agents oxydants, des extraits de plantes, des flavonoïdes et des polyphénols végétaux ou leurs mélanges.

10. Microémulsion de type eau dans l'huile selon l'une des revendications 1 à 9, **caractérisée en ce qu'**elle contient des substances actives solubles dans les graisses qui sont choisies parmi des antioxydants, des vitamines et des provitamines, des acides gras insaturés, des céramides, des huiles essentielles ou leurs mélanges.

11. Microémulsion de type eau dans l'huile selon l'une des revendications 1 à 10, **caractérisée en ce qu'**elle contient des agents dermatologiquement actifs, choisis parmi des hormones et des substances analogues à des hormones, des antimycosiques, des cicatrisants, des tannins, des goudrons, des kératolytiques, des agents de kératoplastie, des photocoumarines, de l'acide azélaïque ou leurs mélanges.

12. Microémulsion de type eau dans l'huile selon l'une des revendications 1 à 11, **caractérisée en ce qu'**elle contient comme additifs des électrolytes, des oxydants, des agents chélatants, des agents renforçant la diffusion, des agents facilitant la pénétration, des agents maintenant le taux d'humidité ou leurs mélanges.

13. Microémulsion de type eau dans l'huile selon l'une des revendications 1 à 12, **caractérisée en ce qu'**elle contient des transporteurs d'oxygène biologiques choisis parmi l'hémoglobine naturelle, modifiée ou non modifiée, la myoglobine, ou leurs mélanges en une quantité totale de 0,001 à 20 % en masse.

14. Microémulsion de type eau dans l'huile selon la revendication 13, **caractérisée en ce qu'**elle contient en outre des antioxydants, du glutathion, une superoxyde-dismutase, de la mélatonine, des flavonoïdes, des aminoacides ou leurs mélanges.

15. Microémulsion de type eau dans l'huile selon l'une des revendications 13 ou 14, **caractérisée en ce qu'**elle contient en outre du glucose.

16. Microémulsion de type eau dans l'huile selon l'une des revendications 1 à 15, **caractérisée en ce qu'**elle contient en outre des vitamines et des provitamines en une quantité de 0,01 à 1,0 % en masse.

17. Microémulsion de type eau dans l'huile selon l'une des revendications 1 à 16, **caractérisée en ce qu'**elle contient des extraits de plantes en une quantité de 0,1 % en masse à 5 % en masse, 0,1 % en masse à 5 % en masse d'huiles essentielles, 0,1 % en masse à 10 % en masse d'acides AHA, 0,01 à 0,3 % en masse d'hormones ou de substances analogues à des hormones, 0,1 à 5 % en masse d'acides gras essentiels, de céramides ou de leurs mélanges.

18. Préparation applicable par voie topique sous forme d'une microémulsion, **caractérisée en ce qu'**elle contient un mélange d'une composition selon l'une des revendications 1 à 17 ou d'un de ses mélanges avec 0,1 à 90 % en masse d'une phase aqueuse, la préparation contenant alors 1 à 50 % en masse de la phase huileuse et représentant une microémulsion de type eau dans l'huile ou huile dans l'eau.

19. Procédé de préparation d'une microémulsion selon l'une des revendications 1 à 18, **caractérisé en ce que** l'on mélange la phase huileuse, dans laquelle sont éventuellement présentes des substances actives solubles dans les graisses, contenant le ou les agents tensioactifs, le ou les phospholipides, les cholestérols, les dérivés de cholestérols, ainsi que le ou les alcools et éventuellement des additifs, avec une phase aqueuse et éventuellement la ou les substances actives solubles dans l'eau, éventuellement des additifs, à des températures de 10 à 30°C, et on transforme la microémulsion de type eau dans l'huile primaire ainsi obtenue éventuellement avec une phase aqueuse qui peut éventuellement contenir d'autres substances actives solubles dans l'eau, éventuellement des additifs, en une microémulsion de type eau dans l'huile secondaire ou une microémulsion de type huile dans l'eau secondaire.

20. Utilisation topique non thérapeutique d'une microémulsion selon l'une des revendications 1 à 18 comme préparation cosmétique pour la peau ou les soins, le lavage, le conditionnement des cheveux.

21. Utilisation selon la revendication 20, **caractérisée en ce qu'**il s'agit d'une peau altérée par l'âge.

22. Utilisation selon la revendication 20 ou 21, **caractérisée en ce que** l'on utilise une préparation selon les revendications 2, 13 à 15.

23. Utilisation selon l'une des revendications 20 à 22, **caractérisée en ce que** l'émulsion est liquide et est appliquée par pulvérisation ou **en ce qu'**elle est appliquée sous forme de gel.

24. Utilisation d'une microémulsion selon l'une des revendications 1 à 18 pour la préparation d'une composition pharmaceutique dermatologique destinée au traitement topique d'une peau dégénérée, abîmée, irritée ou altérée de manière allergique, bactérienne, immunologique ou sous l'effet d'influences extérieures.

25. Utilisation selon la revendication 24, **caractérisée en ce que** l'on utilise une composition selon les revendications 2, 13 à 15.

26. Utilisation selon l'une des revendications 24 ou 25, **caractérisée en ce que** l'émulsion est liquide et appliquée par pulvérisation ou **en ce qu'**elle est appliquée sous forme de gel.
